# EUROPEAN PATENT APPLICATION

(11) **EP 0 918 091 A1**
(43) Date of publication of application: **26.05.1999**
(21) Application number: 97402811.0
(22) Date of filing: 21.11.1997
(51) Int. Cl.: C12N 15/12, C07K 14/47, C07K 16/18, C12Q 1/68, G01N 33/53, A61K 38/17

(54) **A gene called XLIS and the XLIS gene product, called doublecortin and their applications**

(71) Applicant: INSTITUT NATIONAL DE LA SANTE ET DE LA RECHERCHE MEDICALE (INSERM), 75654 Paris Cédex 13 (FR)
(72) Inventor: Chelly, Jamel, 94200 Ivry Sur Seine (FR); Kahn Axel, 75015 Paris (FR); des Portes, Vincent, 75013 Paris (FR); Pinard, Jean-Marc, 78530 BUC (FR)
(74) Representative: Le Guen, Gérard

(57) **Abstract**

The present invention relates to the identification of a new gene, called *XLIS*, and of the *XLIS* gene product, called doublecortin, as well as to the diagnostic and therapeutic applications of these nucleotide and peptide sequences.

SEQUENCE LISTING

(1) GENERAL INFORMATION:

(i) APPLICANT:
(A) NAME: INSERM
(B) STREET: 101, rue de Tolbiac
(C) CITY: Paris
(E) COUNTRY: FRANCE
(F) POSTAL CODE (ZIP): 75013

(ii) TITLE OF INVENTION: New XLIS gene

(iii) NUMBER OF SEQUENCES: 66

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(2) INFORMATION FOR SEQ ID NO: 1:

(i) SEQUENCE CHARACTERISTICS:
(A) LENGTH: 1129 base pairs
(B) TYPE: nucleic acid
(C) STRANDEDNESS: single
(D) TOPOLOGY: linear

(ii) MOLECULE TYPE: DNA (genomic)

(vi) ORIGINAL SOURCE:
(A) ORGANISM: Homo sapiens

(xi) SEQUENCE DESCRIPTION: SEQ ID NO: 1:

CTNTTTTTTT CCCCCAAAAA TTCNAAAATA TTTTTCCCCC TGGNTTGGTT CCAATTCCCA 60

AAATTTCTTT TATTAAANTT GGNGAATTTN CTTTAAAAAA ACNAAAAAAA CCAAGTTGTG 120

GGAAAATTGA GTAAAATCCC TTAAAGGAAT TTGGCAGATT TTATTTNATT TTTTTTTTTT 180

CTCAAGGAGG TAAAAGGAAG AGAGTAACAA ATTTTTAAGG AAGCCTGGGT TGGCTGTTTG 240

GAGTTTGGCC CCCAGGCAGA TTAGGCCAAG GTTTTGGCCA AGTGAAATTG CCAATTTTCT 300

AAAAGAAAGG GCTAGCACAT TGCTCATTAG AGCATTCTGA TTTTGTCTGC GCAATCTTTC 360

TGCTACCCCG CAATTTCCTG TTGGTTATAA ATGAAACCTT TTTAGCTGTT AATGCAGCCT 420

GTGAATTTTT TTAAAAGCAT GTAATTAATC ATAGGAGGTT GGGGGGATTC ACTAAGCCTG 480

AGTTACATGG GAGAAGCTGG ACAAGGCAGT AGGACCTAGA AGGCATCTAT CCACCCTGGC 540

AGGAATTTCT TGCTTGGAGC TCAGACAACA AAGGCATAGA GAGATTGGTT TTCTTTCTCT 600

CAGCATCTCC ACCCAACCAG CAGAAAACCG GTGAGTGGGG CTTTTAAGTG ATTTTCAAGA 660

AGAATGTAAC AGATGTCAAA CGGGAAAAGC ACAAGGCAAA GCCTGCTCTC TCTGTCTCTC 720

TGTCTCCTCT TCTCCTTTTT TGCCTTATTC TATCCGATTT TTTCCCTAAG CTTCTACCTG 780

GGATTTTCCT TTGGAAAAGT GAGTTTGATG TTCCTTTGTT TTCACTGTGA TGTTAATTTA 840

GAATAATACT ACCTCTGATC CTAAAGCAAA GCAAAGCCTT ACTGGCATGC CTGGGGAAAT 900

GTTTGCTGCT TGCCTTGAAG AAGTGGGGTC TCTTACCACT GCAGGTTGTC TGACAGAGAC 960

AATGCTGAGC TCAGCATAGG TCATGGTGAC ATTGGAAAAA AGGCGGAATT GAGCCTGGCC 1020

AGACCCATTA NGCACCAGTC TTTCTTATCT CCTGTCCTCC TGGTCCCCTT GCAAATATAT 1080

TGATGTTGCC ATGTTTTACC ANCTNAACCC TGCNTTGCCT TTGNNAATN 1129

(2) INFORMATION FOR SEQ ID NO: 2:

(i) SEQUENCE CHARACTERISTICS:
(A) LENGTH: 1654 base pairs
(B) TYPE: nucleic acid
(C) STRANDEDNESS: single
(D) TOPOLOGY: linear

(ii) MOLECULE TYPE: DNA (genomic)

(vi) ORIGINAL SOURCE:
(A) ORGANISM: Homo sapiens

(xi) SEQUENCE DESCRIPTION: SEQ ID NO: 2:

CACAATTAAA TTTTTTCCCA TTAAGGAGGT GTTNNTNGCA TTCAATTGGG GNAGGGGGTT 60

GGACCAACNT GGGGGGGGAA AAAAAAGGAT TTTTGTGAAA CAAAATGGGA ACCNGGGGGA 120

AGACAAGAGT TAGTAAACTT GTTAAATAAA CTTATTTTTT CTAATCCCTT TTTTCCCCCC 180

AGCTTATTTT TTATGAATGT CGGATAGCTG CACCAGCTTG GTGGGGAAAG GGTTTGATGA 240

ATAGCACAAA GACACTGGCT GTTCCCTGGA GGCTGTCCCT TTAAAGGAGA ATCTTAGTTT 300

ATTCTGGGGG GAGGGGATGC ACACATTAGA GTAGGAAAGA GGGCTTGGAA TAAAATGAAA 360

ACACTCCCCC TTCATAGTCA TTGTACTGAA ATGCAAAGAC TGCTTCCTAA GCTGGAGATG 420

CTAACCTTGG GTAGCTCCTT CTGTTCTCTT CAAGGGGAAT TTTGTCAGGC TATGGATTCA 480

TTTACAACTG TTAGTCATGT GGGCATGTGT GAGGAAACAG ATGCCAGTTT TAATGTATTT 540

AGCCCGAAGT TCCAATTTGA TAGGAGCCAC TGTCAGTAAG TCTCAGGATT TTCAGCTATT 600

TCAAAATCTC CCCTTCTCCT CTGTCTGGAA CAGTGCCAAG AGTGCCTCCC TCTCTATCTC 660

TTACTCCCAA CCCCCACAAC CACCAGCACC CCCGCCCAGC CCCTCCTTCT TCTCTATTAA 720

GATCAATATT CCTGCAGGTC AGGGGCAAGC AGCAGATGGG TCACAGCTTT TTTCAACCAT 780

TCTTTTCCAC AAGCAGCAGA TTGCAATCCT GGATCTTGGC TAATATTTAA AAATCCCTTC 840

TTTTTTCCCT TCTCCTTGTC CTTTTTGTTT TTGCCTCTCT TCACCCCCAT CCCTTTCTCC 900

CACGCTCAGG TCTCTGAGGT TCCACCAAAA TATGGAACTT GATTTTGGAC ACTTTGACGA 960

AAGAGATAAG ACATCCAGGA ACATGCGAGG CTCCCGGATG AATGGGTTGC CTAGCCCCAC 1020

TCACAGCGCC CACTGTAGCT TCTACCGAAC CAGAACCTTG CAGGCACTGA GTAATGAGAA 1080

GAAAGCCAAG AAGGTACGTT TCTACCGCAA TGGGGACCGC TACTTCAAGG GGATTGTGTA 1140

CGCTGTGTCC TCTGACCGTT TTCGCAGCTT TGACGCCTTG CTGGCTGACC TGACGCGATC 1200

TCTGTCTGAC AACATCAACC TGCCTCAGGG AGTGCGTTAC ATTTACACCA TTGATGGATC 1260

CAGGAAGATC GGAAGCATGG ATGAACTGGA GGAAGGTAAT TTAAATAGTG GGTGGTGGCC 1320

GTTGGTGGGA GGTGGCATCA TTGGTTATGG TTACATTCTT CGGTTGCTTT GAAAAAAAAT 1380

TAGGCAATGT ATTTTTCAAA ACACCGGGTT GATTGATGCT CAAATTTCTA ATGCTATAGA 1440

CATCAACAGA ACATTAGCAA TCACCTTCCA TCTCTGCTGA AGTTGAAATT GTGAATGGTG 1500

GTGAATCATG CCTTTGTGCC TATCTGCCCA GGTTTTNAAA AAGAAATCTT AATTGTTACC 1560

ATTACTCCCA AATTTGTTCC CTAATTACNC CNATTTCCCT ATTGAGGGNA ACCTTAATCC 1620

TTAGGTTTTA CNAAAAAGAA NTTGGTTGGT TTTT 1654

(2) INFORMATION FOR SEQ ID NO: 3:

(i) SEQUENCE CHARACTERISTICS:
(A) LENGTH: 1284 base pairs
(B) TYPE: nucleic acid
(C) STRANDEDNESS: single
(D) TOPOLOGY: linear

(ii) MOLECULE TYPE: DNA (genomic)

(vi) ORIGINAL SOURCE:
(A) ORGANISM: Homo sapiens

(xi) SEQUENCE DESCRIPTION: SEQ ID NO: 3:

TGTATAACCC AGTGGGGAAG GGGNAGGTGC AAGGGGATAA GTGGGTAACG CAGGGTTTCC 60

AAGTCAGGAA GTGTAAAAAG GACGGCAGTG GAATGTAATA NGAATCAATA TAAGGGGGAA 120

TGGGAGTCCA CCGCGGTGGC GGCNGTTCTA GNAATTAGTG GAATCCCCCG GGGTGCAAGG 180

AATTCGATAT CAAGCTTGGA ACTCCAACTT GTTATATTAA ATTTAGATAT GCTCTTTGTC 240

CTTAGAACCC TTAGCTTTAC AGAGTTCTTC AACTAGGTGT GTCATTATAT TGAAGGATGT 300

TTTGAGAATT ACTCTTGAAT TTGGATGAGC CAAGTTTTTA GCCCAATTAG GAATTGCACC 360

TAAGGTTGGT TAGTATCACC ATTTATTGAA AAGTTCTACT CCAGTGTCAG TGTGTATTAG 420

TTTGGTTAGA ATAATCCATA TATCTGCTAT TAAACTAATC ACCTAATCAC TTATTTCTTG 480

CCTTAGGGGA AAGCTATGTC TGTTCCTCAG ACAACTTCTT TAAAAAGGTG GAGTACACCA 540

AGAATGTCAA TCCCAACTGG TCTGTCAACG TAAAAACATC TGCCAATATG AAAGCCCCCC 600

AGTCCTTGGC TAGCAGCAAC AGTGCACAGG CCAGGGAGAA CAAGGACTTT GTGCGCCCCA 660

AGCTGGTTAC CATCATCCGC AGTGGGGTGA AGCCTCGGAA GGCTGTGCGT GTGCTTCTGA 720

ACAAGAAGAC AGCCCACTCT TTTGAGCAAG TCCTCACTGA TATCACAGAA GCCATCAAAC 780

TGGAGACCGG GGTTGTCAAA AAACTCTACA CTCTGGATGG AAAACAGGTA GGTACTTTTT 840

CAAAGTACTT TTCCCGTTTT TTCTAAATTC TTAGATGATC CGTTGACCTG CAGGTCGACC 900

TCGAGGGGGG GCCCGGTACC AGCTTTTGTT CCCTTTAGTG AGGGTTAATT TCGAGCTTGG 960

CGTAATCATG GTCATAGCTG TTTCCTGTGT GAAATTGTTA TCCGCTCACA ATTCCACACA 1020

ACATACGAGC CGGAAAGCAT AAAGTTGTTA AAGCCTGGGG TTGCCTAATG AATTGANCTA 1080

ACTCCACATT AATTGCGTTT GCGCTCCACT GCCCCGCTTT CCCATTCCGG GAAACCTGTT 1140

CCGTGCCCAC CTGCCATTTA AATGATCCCG CCAACGCGCC GGGAAAAAAG CCGGTTTGCG 1200

TTTATTGGCG CTCTTCCCCT TNCCTCCGCT NAATAAATCC CCTGCNCTCG GTNCNTTTCC 1260

GNTTGCGGGA AACCGGTTTT CACC 1284

(2) INFORMATION FOR SEQ ID NO: 4:

(i) SEQUENCE CHARACTERISTICS:
(A) LENGTH: 816 base pairs
(B) TYPE: nucleic acid
(C) STRANDEDNESS: single
(D) TOPOLOGY: linear

(ii) MOLECULE TYPE: DNA (genomic)

(vi) ORIGINAL SOURCE:
(A) ORGANISM: Homo sapiens

(xi) SEQUENCE DESCRIPTION: SEQ ID NO: 4:

TCAGCTACAG TCAGGTGCAG AAGATTTTTC TCATCTTCCT TGGCCCCAGT CCCTTGCCCT 60

AGAGNNGGGC CCAGGAGCTT CCCGTCTTTG AGGCTTCTGA GAAAAGGTGA CATTTATAGG 120

GCCATCTGCT GGTTGACTTT TCTTCAGAAA ACGGCATTCC ATTTGGACAT GGTCTCAGTC 180

TTACTCTTGA TGCAATTGAA AGTACCATAC ATGAAACATG GATAGACAAT GGTACTCAGT 240

ATATCATCCC TCACTAGGGG TCATGATTCA GACTATGAAT GTGGTAGGTG TTTTAAGAAA 300

GAGAAAACTC CTGGGAGCTG AAGATGATGG CCTAGATGGG AAGCTTTTCT CCTTGTCAGT 360

GTAGTGTGAG GTTCATTGTC ACAGGACCAT CATATACAAT CAGTGTTTCT GCTTTCTCCA 420

TAGGTAACTT GTCTCCATGA TTTCTTTGGT GATGATGATG TGTTTATTGC CTGTGGTCCT 480

GAAAAATTTC GCTATGCTCA GGATGATTTT TCTCTGGATG AAAATGGTAA GCATAACCAC 540

TGGGTTTTAT TGCTCCATTG TTCTCTCCCC TTCTATCCTT TAGGATTTCC ATGGGTTTGT 600

ATGGTGTGGA AAACATCTAT GCATGATCCT TCAAGGGCTT ATAATGTTGG TCTTTCTCCT 660

GTATATTTGG ATACATGGGT CATGACCCAT GTAAGACTGA AAGACTGACT GGATATCCCA 720

GAGTCCCAGG CTCCAAACTC AATAAAACTC ACTATCTGCC CCAATAGTGA CCATATTTCT 780

TGAACCATAT ATTGTGTCAC ATGGGATAAC AGGCAC 816

(2) INFORMATION FOR SEQ ID NO: 5:

(i) SEQUENCE CHARACTERISTICS:
(A) LENGTH: 1069 base pairs
(B) TYPE: nucleic acid
(C) STRANDEDNESS: single
(D) TOPOLOGY: linear

(ii) MOLECULE TYPE: DNA (genomic)

(vi) ORIGINAL SOURCE:

(A) ORGANISM: Homo sapiens

(xi) SEQUENCE DESCRIPTION: SEQ ID NO: 5:

AAACTTTTCC NTTCAAAAAA AATTCNANGG ANGTTTTCAT CGGGTAGCNG GTAACTTCNG 60

GAGTGGNTAA TNTCTAATTC AGGGAAATTA ATTAAACATT ATATCAATAG TGCTATTCTG 120

AGACATAGNT AGCTTGTCCA TAAANGATGG AATNGACTAT TTAATNGCNN ACTNGACCTG 180

TTTATGGATT CTNGCCCTNG TTTTCGTAAT TCAGGGGCTT AAACACCTAG CTTAGCCCAG 240

GTGGCTTTTG AAGGTTTCCC TAATGCCTCC CTCCCCAGAA CCATGGCTCC TACTAATAAT 300

AAGGACCACA TTGTAGTCCT GACCCATTTA GGTCCTTTGG CCTGTGATGG TTATTGCGGT 360

TTCCAGAGGC TGATAACATG CTGAGCCTGT TTTATCCTCT ACTAAGCTGT CTGTGTCCTT 420

TTGCCCCAGA ATGCCGAGTC ATGAAGGGAA ACCCATCAGC CACAGCTGGC CCAAAGGCAT 480

CCCCAACACC TCAGAAGACT TCAGCCAAGA GCCCTGGTCC TATGCGCCGA AGCAAGTCTC 540

CAGCTGACTC AGGTAACGAC CAAGACGGTG AGTGCTCTTT TCCTAACTGT GCACGCTGAC 600

TTCATTTATG GAGGACAATA CTTTCTGCAT GCAGAGGAAT CAGTTCCTCA TGAACACCAC 660

TGTGTCTCCA TGAAACCCTA TTCTATCAAT TCAGGGACTA AAACAGTCAA ACTTGTTTGG 720

AGTCATGGAG GCACAGCTAC AACCATTATT CCATTCAAAT GGATGCAAAC CAGAAGCCTG 780

GCCCATTGTC TATGCTTGCT GTAATGTTTA TTTTCATGGT TGTCATCATT TTCATCATCT 840

TCAACATCAT GTGGCAAAAA CATATTCAAT GGAATAATCA AATTAACCAT AAACCTGAAA 900

TTAAAAATCC GAATCNNGAA AAAAGAATAT TANAAACNAC ACCCCCANAG TGTCCACATA 960

GTTGAATCAC TTGAACNTCC AATTTGAATC CGAAATTATT TAAAACCCCN AACCAAAANG 1020

GAAACNAGTT CCCTACNTTT TTTTGCCCTT TTATTNGGGG ANANAAAAA 1069

(2) INFORMATION FOR SEQ ID NO: 6:

(i) SEQUENCE CHARACTERISTICS:
(A) LENGTH: 617 base pairs
(B) TYPE: nucleic acid
(C) STRANDEDNESS: single
(D) TOPOLOGY: linear

(ii) MOLECULE TYPE: DNA (genomic)

(vi) ORIGINAL SOURCE:
(A) ORGANISM: Homo sapiens

(xi) SEQUENCE DESCRIPTION: SEQ ID NO: 6:

AAGCTTTACC TATGGGACTG ACAGGAACAC TATCAGNCCA TCTTTCTGCT TAATGTTTTC 60

AATTTTCTAT TTATTCTCTC TGTCTCCCTC CCCCCCACTC TTTCTCCCTC CCCCTCTCTC 120

TTCCTCTCCC CCACTCTCCA CACACCCTCA GGATATTTGC CTTTGTTTTT AAAGCACATT 180

TTAGAATATC TCTGTTTAAC AATTTAGGTC TTAAGATTAA AAAAAAAATA CTTTAGTATG 240

AAGTGTCTGA ACAAGAATCC ACTTTGAAAG CTGTTTAATT GTTCCATTTT AATATTCCAC 300

TTTTCTTTCT TCATGATTGG ATATTAAAAA GCTCATTAGC AAGGGAATAT AAAACAATTT 360

AGCAACACNA TAGAATATAA AGATGTTTAA AAAGAAATCT GATTTTCCAG CATTTCCTTG 420

GCTAAACCCA ATGTTGGTGA ACCCTGACTC CNGCTACCAT TTNGATCTTT AGTGTCTCTA 480

TGCCTTCTCC TGTTTCAACT CCCCCTCTCT TTTAATCTAC ACTCCTGTCT CTTCTCCCCA 540

TCCCACTCTT GTCTCNCCCT TTTAATCNCC CCGCCATCNG CGCTACCTCC TTAGTCCTCG 600

TCCCCCAGTC TCTCTCC 617

(2) INFORMATION FOR SEQ ID NO: 7:

(i) SEQUENCE CHARACTERISTICS:
(A) LENGTH: 741 base pairs
(B) TYPE: nucleic acid
(C) STRANDEDNESS: single
(D) TOPOLOGY: linear

(ii) MOLECULE TYPE: DNA (genomic)

(vi) ORIGINAL SOURCE:
(A) ORGANISM: Homo sapiens

(xi) SEQUENCE DESCRIPTION: SEQ ID NO: 7:

ATGGGGGTTT TTTTAACNAA CCATTATTGG GCACNAAGGG AATGGCAATG GTCCAAATNG 60

CCATATTTTT TCCCTTTAAG GTAAGGCACA TAAACTTTTC TGTGCTTCAA TTTTGTCTTC 120

TATAAAATTA AAGCAAAATA ATACTTTCCC AGATTACTTC CCAGAGATGT TAGGGGCAAA 180

CCTGGGAATG AATATGAAAA ATGTTTGAAA AGAAACAGAC CCCTGTGATT GGGAAGCACT 240

GAGGAGGAAA GGGTGGGAGG ATGACGGCCT TGCACTGCAT AGGGATCCAA TTAACTGAGG 300

ATTGCAGTTC TTGCCCTTTT GAGGAATGTG TTTCTTTTCA CCTTTGTTCT ACTCACCAAA 360

TCACTTGGTT GTCTGTGTAC CTTGTTTGAA TGTTCTTAGT GTCTTCTGAG GGGAAGGATA 420

ACTTGCTCCT TTGTATGCTG TTGATTTTTA TCCCTTCCTT TTCTCTTGCT TTGGGCTAGC 480

AAACGGAACC TCCAGCAGCC AGCTCTCTAC CCCCAAGTCT AAGCAGTCTC CCATCTCTAC 540

GCCCACCAGT CCTGGCAGCC TCCGGAAGCA CAAGGTATTA TGTCTCTTAT ACCTTACTAA 600

ACCTCTTTGC ACTCAGTTTC CTGAATGGCT TGGTGAAGGT TTTCTCTTCC ATTCTAACTC 660

AACAGCACAT TAAGGCCAGA AATTTTCTTC ATCTGGCTTC ATTAGCTTGA AGTTCTGCGA 720

ATCCAAGCAG GGAGCAAGCT T 741

(2) INFORMATION FOR SEQ ID NO: 8:

(i) SEQUENCE CHARACTERISTICS:
(A) LENGTH: 489 base pairs
(B) TYPE: nucleic acid
(C) STRANDEDNESS: single
(D) TOPOLOGY: linear

(ii) MOLECULE TYPE: DNA (genomic)

(vi) ORIGINAL SOURCE:

(A) ORGANISM: Homo sapiens

(xi) SEQUENCE DESCRIPTION: SEQ ID NO: 8:

AAGCTTTTGG TTGATTTTTA GAGTCCTGAA AAAGTTAATT TTGACAGTTT TGCCAGTGTT 60

ACTGCATTTG TTGAGGAACA GATTTTCAGA GGGGCTTATG CCACCTTTCT GGAAGTCCAG 120

AGCTCAAAAA TACTTTTGAC TATGTTCACT TTACTCCAGT ACTTCAGAAT TAATCCCTTC 180

AGAGATGCAA CTCCATTGTA TGCCACTTTG CTTCAATTCC ATTTTACATG TATTTGTTGC 240

ATTTCTAGTA TTTCCTTGGC ATTGTACATC CTACCTGTTT ATCAGTTTTC TAAGAAACTT 300

TTGTTTGGTA GTTTAGAACA GGGATGCCAG ATCCTGCCTG TGTCTTGGTT ATATTTGCAT 360

GGATGACCCA CCCCATCTAA AATATGTTTT TTTAAATATT TAAAGAATTG TAAAAGANCN 420

AACCCCCAAC NAATNATGAA GAATGTGTGA CAGAAACCNT ATGTGACCAG CCAAGAAAAC 480

TTTANATTG 489

(2) INFORMATION FOR SEQ ID NO: 9:

(i) SEQUENCE CHARACTERISTICS:
(A) LENGTH: 755 base pairs
(B) TYPE: nucleic acid
(C) STRANDEDNESS: single
(D) TOPOLOGY: linear

(ii) MOLECULE TYPE: DNA (genomic)

(vi) ORIGINAL SOURCE:
(A) ORGANISM: Homo sapiens

(xi) SEQUENCE DESCRIPTION: SEQ ID NO: 9:

AGATTATTTG TTGCCNGGGT TCAGGGGAAG AGGGGAGGTT GGATGGAGTT TTAATGGGGT 60

AGCACAAGGG GGCCTTGTGT TGAAGGTACA GTTCTGTATC TTGACTGTGG CGATGGTCAC 120

ACAAATATAC ACACATGATA AAATTGCATA GGNATATACA CACACACACA TACACACCCC 180

ACACACAAAT GATCACATGT TAAACTGCAA GATGGTACCA TTAGGGAAAA TTGAATGAAG 240

GGTACATTGG TACAAGGGTA CCTCCCTGTA CATTTTTTCA ACTTCCTGTG AATCTATAAT 300

TATTATGTTG TAAAAATTAA AGTATTAATA AAAAAAACTA AAGCAGACAT TCCAGAGCTC 360

AAGATATCAA GAAAAGGAAA ATTAACTTTG TCTCTTCTCT TCTTATAGGA CCTGTACCTG 420

CCTCTGTCCT TGGATGACTC GGACTCGCTT GGTGATTCCA TGTAAAGGAG GGGAGAGTGC 480

TCAGAGTCCA GAGTACAAAT CCAAGCCTAT CATTGTAGTA GGGTACTTCT GCTCAAGTGT 540

CCAACAGGGC TATTGGTGCT TTCAAGTTTT TATTTTGTTG TTGTTGTTAT TTTGAAAAAC 600

ACATTGTAAT ATGTTGGGTT TATTTTCCTG TGATTTCTCC TCTGGGCCAC TGATCCACAG 660

TTACCAATTA TGAGAGATAG ATTGATAACC ATCCTTTGGG GCAGCATTCC AGGGATGCAA 720

AATGTGCTAG TCCATGACCT TTCAATGGAA AGCTT 755

(2) INFORMATION FOR SEQ ID NO: 10:

(i) SEQUENCE CHARACTERISTICS:
(A) LENGTH: 38 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 10:

CTTTCTCTCA GCATCTCCAC CCAACCAGCA GAAAACCG 38

(2) INFORMATION FOR SEQ ID NO: 11:

(i) SEQUENCE CHARACTERISTICS:
(A) LENGTH: 109 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 11:

CACAAGGCAA AGCCTGCTCT CTCTGTCTCT CTGTCTCCTC TTCTCCTTTT TTGCCTTATT 60

CTATCCGATT TTTTCCCTAA GCTTCTACCT GGGATTTTCC TTTGGAAAA 109

(2) INFORMATION FOR SEQ ID NO: 12:

(i) SEQUENCE CHARACTERISTICS:
(A) LENGTH: 393 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 12:

CTTATTTTTT ATGAATGTCG GATAGCTGCA CCAGCTTGGT GGGGAAAGGG TTTGATGAAT 60

AGCACAAAGA CACTGGCTGT TCCCTGGAGG CTGTCCCTTT AAAGGAGAAT CTTAGTTTAT 120

TCTGGGGGGA GGGGATGCAC ACATTAGAGT AGGAAAGAGG GCTTGGAATA AAATGAAAAC 180

ACTCCCCCTT CATAGTCATT GTACTGAAAT GCAAAGACTG CTTCCTAAGC TGGAGATGCT 240

AACCTTGGGT AGCTCCTTCT GTTCTCTTCA AGGGGAATTT TGTCAGGCTA TGGATTCATT 300

TACAACTGTT AGTCATGTGG GCATGTGTGA GGAAACAGAT GCCAGTTTTA ATGTATTTAG 360

CCCGAAGTTC CAATTTGATA GGAGCCACTG TCA 393

(2) INFORMATION FOR SEQ ID NO: 13:

(i) SEQUENCE CHARACTERISTICS:
(A) LENGTH: 386 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 13:

GTCTCTGAGG TTCCACCAAA ATATGGAACT TGATTTTGGA CACTTTGACG AAAGAGATAA 60

GACATCCAGG AACATGCGAG GCTCCCGGAT GAATGGGTTG CCTAGCCCCA CTCACAGCGC 120

CCACTGTAGC TTCTACCGAA CCAGAACCTT GCAGGCACTG AGTAATGAGA AGAAAGCCAA 180

GAAGGTACGT TTCTACCGCA ATGGGGACCG CTACTTCAAG GGGATTGTGT ACGCTGTGTC 240

CTCTGACCGT TTTCGCAGCT TTGACGCCTT GCTGGCTGAC CTGACGCGAT CTCTGTCTGA 300

CAACATCAAC CTGCCTCAGG GAGTGCGTTA CATTTACACC ATTGATGGAT CCAGGAAGAT 360

CGGAAGCATG GATGAACTGG AGGAAG 386

(2) INFORMATION FOR SEQ ID NO: 14:

(i) SEQUENCE CHARACTERISTICS:
(A) LENGTH: 341 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 14:

GGGAAAGCTA TGTCTGTTCC TCAGACAACT TCTTTAAAAA GGTGGAGTAC ACCAAGAATG 60

TCAATCCCAA CTGGTCTGTC AACGTAAAAA CATCTGCCAA TATGAAAGCC CCCCAGTCCT 120

TGGCTAGCAG CAACAGTGCA CAGGCCAGGG AGAACAAGGA CTTTGTGCGC CCCAAGCTGG 180

TTACCATCAT CCGCAGTGGG GTGAAGCCTC GGAAGGCTGT GCGTGTGCTT CTGAACAAGA 240

AGACAGCCCA CTCTTTTGAG CAAGTCCTCA CTGATATCAC AGAAGCCATC AAACTGGAGA 300

CCGGGGTTGT CAAAAAACTC TACACTCTGG ATGGAAAACA G 341

(2) INFORMATION FOR SEQ ID NO: 15:

(i) SEQUENCE CHARACTERISTICS:
(A) LENGTH: 103 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 15:

GTAACTTGTC TCCATGATTT CTTTGGTGAT GATGATGTGT TTATTGCCTG TGGTCCTGAA 60

AAATTTCGCT ATGCTCAGGA TGATTTTTCT CTGGATGAAA ATG 103

(2) INFORMATION FOR SEQ ID NO: 16:

(i) SEQUENCE CHARACTERISTICS:
(A) LENGTH: 123 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 16:

AATGCCGAGT CATGAAGGGA AACCCATCAG CCACAGCTGG CCCAAAGGCA TCCCCAACAC 60

CTCAGAAGAC TTCAGCCAAG AGCCCTGGTC CTATGCGCCG AAGCAAGTCT CCAGCTGACT 120

CAG 123

(2) INFORMATION FOR SEQ ID NO: 17:

(i) SEQUENCE CHARACTERISTICS:
(A) LENGTH: 95 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 17:

CAAACGGAAC CTCCAGCAGC CAGCTCTCTA CCCCCAAGTC TAAGCAGTCT CCCATCTCTA 60

CGCCCACCAG TCCTGGCAGC CTCCGGAAGC ACAAG 95

(2) INFORMATION FOR SEQ ID NO: 18:

(i) SEQUENCE CHARACTERISTICS:
(A) LENGTH: 57 base pairs
(B) TYPE: nucleic acid
(C) STRANDEDNESS: single
(D) TOPOLOGY: linear

(ii) MOLECULE TYPE: cDNA

(vi) ORIGINAL SOURCE:

(A) ORGANISM: Homo sapiens

(xi) SEQUENCE DESCRIPTION: SEQ ID NO: 18:

GACCTGTACC TGCCTCTGTC CTTGGATGAC TCGGACTCGC TTGGTGATTC CATGTAA 57

(2) INFORMATION FOR SEQ ID NO: 19:

(i) SEQUENCE CHARACTERISTICS:
(A) LENGTH: 9417 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 19:

CTTATTTTTT ATGAATGTCG GATAGCTGCA CCAGCTTGGT GGGGAAAGGG TTTGATGAAT 60

AGCACAAAGA CACTGGCTGT TCCCTGGAGG CTGTCCCTTT AAAGGAGAAT CTTAGTTTAT 120

TCTGGGGGGA GGGGATGCAC ACATTAGAGT AGGAAAGAGG GCTTGGAATA AAATGAAAAC 180

ACTCCCCCTT CATAGTCATT GTACTGAAAT GCAAAGACTG CTTCCTAAGC TGGAGATGCT 240

AACCTTGGGT AGCTCCTTCT GTTCTCTTCA AGGGGAATTT TGTCAGGCTA TGGATTCATT 300

TACAACTGTT AGTCATGTGG GCATGTGTGA GGAAACAGAT GCCAGTTTTA ATGTATTTAG 360

CCCGAAGTTC CAATTTGATA GGAGCCACTG TCAGTCTCTG AGGTTCCACC AAAATATGGA 420

ACTTGATTTT GGACACTTTG ACGAAAGAGA TAAGACATCC AGGAACATGC GAGGCTCCCG 480

GATGAATGGG TTGCCTAGCC CCACTCACAG CGCCCACTGT AGCTTCTACC GAACCAGAAC 540

CTTGCAGGCA CTGAGTAATG AGAAGAAAGC CAAGAAGGTA CGTTTCTACC GCAATGGGGA 600

CCGCTACTTC AAGGGGATTG TGTACGCTGT GTCCTCTGAC CGTTTTCGCA GCTTTGACGC 660

CTTGCTGGCT GACCTGACGC GATCTCTGTC TGACAACATC AACCTGCCTC AGGGAGTGCG 720

TTACATTTAC ACCATTGATG GATCCAGGAA GATCGGAAGC ATGGATGAAC TGGAGGAAGG 780

GGAAAGCTAT GTCTGTTCCT CAGACAACTT CTTTAAAAAG GTGGAGTACA CCAAGAATGT 840

CAATCCCAAC TGGTCTGTCA ACGTAAAAAC ATCTGCCAAT ATGAAAGCCC CCCAGTCCTT 900

GGCTAGCAGC AACAGTGCAC AGGCCAGGGA GAACAAGGAC TTTGTGCGCC CCAAGCTGGT 960

TACCATCATC CGCAGTGGGG TGAAGCCTCG GAAGGCTGTG CGTGTGCTTC TGAACAAGAA 1020

GACAGCCCAC TCTTTTGAGC AAGTCCTCAC TGATATCACA GAAGCCATCA AACTGGAGAC 1080

CGGGGTTGTC AAAAAACTCT ACACTCTGGA TGGAAAACAG GTAACTTGTC TCCATGATTT 1140

CTTTGGTGAT GATGATGTGT TTATTGCCTG TGGTCCTGAA AAATTTCGCT ATGCTCAGGA 1200

TGATTTTTCT CTGGATGAAA ATGAATGCCG AGTCATGAAG GGAAACCCAT CAGCCACAGC 1260

TGGCCCAAAG GCATCCCCAA CACCTCAGAA GACTTCAGCC AAGAGCCCTG GTCCTATGCG 1320

CCGAAGCAAG TCTCCAGCTG ACTCAGCAAA CGGAACCTCC AGCAGCCAGC TCTCTACCCC 1380
CAAGTCTAAG CAGTCTCCCA TCTCTACGCC CACCAGTCCT GGCAGCCTCC GGAAGCACAA 1440
GGACCTGTAC CTGCCTCTGT CCTTGGATGA CTCGGACTCG CTTGGTGATT CCATGTAAAG 1500
GAGGGGAGAG TGCTCAGAGT CCAGAGTACA AATCCAAGCC TATCATTGTA GTAGGGTACT 1560
TCTGCTCAAG TGTCCAACAG GGCTATTGGT GCTTTCAAGT TTTTATTTTG TTGTTGTTGT 1620
TATTTTGAAA AACACATTGT AATATGTTGG GTTTATTTTC CTGTGATTTC TCCTCTGGGC 1680
CACTGATCCA CAGTTACCAA TTATGAGAGA TAGATTGATA ACCATCCTTT GGGGCAGCAT 1740
TCCAGGGATG CAAAATGTGC TAGTCCATGA CCTTTCAATG GAAAGCTTAG GGGCCTGGGG 1800
TAAATTTGCC CCGTTTAAAT TTGCCCAAAC AGTTTTCCTT TTGTAGAGGG GTGTTTAAAT 1860
ATACAGCAAT TAAAAAGTTT GTGTGGGGAA AAAAAAAACT CATTGGCAGA TCCAAGAATG 1920
ACAAACACAA GTGCCCCTTT TCTCTGGATC TCAAGAATGG TGGAGGACCC TGGAAGGACA 1980
GCAAGGCAGC TCCCCAGCCT CACTCTTCAC TCCTGATTGA GGCCCGGGTT TGTTGTCCAG 2040
CACCAATTCT GGCTGTCAAT GGGGAGAAAT AAACCAACAA CTTATAATTG TGACACCAGA 2100
TGCTTAGGAT CCTGGTGCTG GGTTAGCTAA GAGAATAGAC AGAATTGGAA AATACTGCAG 2160
ACATTTCCGA AGAGTTTATA AAGCACAGTG AATTCCTGGT CAATCTCTCC ACTGAGGCAA 2220
TTTGGAATCA ATAAGCAATT GATAATAGTT TGGAGTAAGG GACTTCATAT ACCTGATTCC 2280
TCTAGAAGGC TGTCTAACAT ACCACATGAT TACATGAACT GTATGGTATC CATCTATCTC 2340
TGTTCTATTG AATGCCTTGT TAACAGCCAA CACTGAAAAC ACTGTGAGAA TTTGTTTTCA 2400
GGTCTGACAC CTTTCAGTCT CTTTTTATAG CAAGAAATCA ATATCCTTTT TATAAAAATT 2460
CATGTCTGTA TTTCAGGAGC AAACTCTTCA GGCTCCTTTT TTATAAACTG GTGATTTTTC 2520
TTTTGTCTAA AAAACACATG AAGAAAATTT ACCAGAAAAA AAAAAAAAAG CCGAAGAATA 2580
ATGTTATTTA GAAATTATGC TGTCACTGCC AAACAGTAAC CTCCAGGAGA AAACAAGATG 2640
AATAGCAGAG GCCAATTCAA TAGAATCAGT TTTTTGATAG CTTTTTAACA GTTATGCTTG 2700
CATTAATAAT TTCAATGTGG ACCAGACATT CTAATTATAT TTTAAATGAA ATGTTACAGC 2760
ATATTTTAAG CAACTCTTTT TATCTATAAT CCTAATATTT CATACTGAAG ACACAGAAAT 2820
CTTTCACTTG TCTTTAACAT TAGAAAGGAT TTCTCTTTAC TAAGGACTGA TCATTTGAAA 2880
TAGTTTTCAG TCTTTTGAGA TACAGGTTTA TAACACTGCT TTTTTTTTCC TGTAAACATA 2940
GCCCATAATG GCAAAAACAA CTAATTTTAA TTGAAGGTCT TGCTTGCCAN TCCTGTGTTG 3000
GCTTTNACCA AATATAAAAA TTCCCTTATT CCTTGGTAAT GGTGCAAATN TTTGGAAAGG 3060
CACAGCATCC AAACCAAGCT GCTGTTTGGC TACTGAATGG CTTGCAGTTG TTCCTCCACT 3120
CTAAATGGAA TGAGCTTGCT GTGTGTGTGT GTGGTGGTGG TGGGAGGGGG TGGTGCATGT 3180
GTGTGTGTGT GTGTGCATCT GCAGCTGCTT CAAAATTAAG AAATACTACA AGACACCCCT 3240
GTAATGGATT GGTGGCAACT GGGTGGCACT GCTGATGTGC ACTGTGTAGG GGGGAACCCA 3300

GTGGTGGTGG GGTATCTCAA ATGCCCCTAG ACAAGCTTCA GATGTCTGTA GCTACCAAAA 3360
ACATTTTCGG TTCAAGAAAA GTGAGATGAT GGTAGTACTG GTTTCTGGTG AAATTGAAAA 3420
ACCCCAAATG ATGAGGATCT CTTTTTGCCC CCTCTCCTTT TTTTGTAAAC CCATTCAAAA 3480
CCATTAATAA GCCCATTTTA CTAANCCCCT ATTTCTTTCT AGAAGCTCAG GGTTTNCTTA 3540
GTGCCTCCCA NAACATTTTG TAGTTAATTG GGAAAAAGTG ATACTTGGAT TAGGGGGTGT 3600
GGGCATAAAG AATGGTGGGA GGCCTGATTT TAAAATTCAG GCCAGAACCC CCAATGACTC 3660
CACCCATAGT NTCACTTTAG GTCTCATTTA GTCCATCACC TTTATTTTAA GTTGAGGAAG 3720
TGGAGGCTGG TAAAGAGCAG GACCAGAGGA AGAATCCAGA TTTCCTTATG CTTGGGCCTC 3780
ACACTAGCTC TNTGAGTATT TCCTTGATTG CGGTATATGT ACTACTAGAA AATACCAAAT 3840
GGATATATTT TCTTTAGGAT AACCTTTGAA CCAACAATNT TCAATAACAA TAGTACATCT 3900
TCCATCTTAC TTTTAATCGA GTATAAGGAA ATGTTTCTTT ATGGCCATTT TGGAGGGAGC 3960
AGGGGATGAG GCTTGGCATA GTCCAAAATT TAAGNCTCCA ATAATTAATT GCATTTTAAA 4020
TTGTTTTAAA TTGGCCCACT TTCAAGGCAA TTTTTTTTGT GTGTCTGTAA CTGAGCTCCT 4080
CCACCCCTGT CATTCACTTC CAATTTTACC CAATCCAATT TTAGCACTCA AGTTCCATTG 4140
TGTTAATTTT TGCACGGTCT ACACACATCA AGTCAGCAAG CATTTGCCAC CACTCCCTAT 4200
ACTTCTCCCT CTTTTTTACA CACACACACA CACACACACA CACAATCCAT CTCTTGCTTG 4260
TTCCTACCTC CCTGATTTTT CTTCCCTACA GAAATAGAAA TAGGGACAAA GAAGGGGAAA 4320
ATGTATATAT TGGGGCTGGG CTGAACAACT AACTTCATAA GTAGTATTAA CTAGGGGTAA 4380
ATTGAGAGAA AAGCTCCTTT TCTCTTCACT GTTTTGGAAA GGATAGCCAT TAGCATGACT 4440
GCTTTGTGTC CTTATGGACT TTAGTATTAG CCTAGATTGA ATTATAGCGT TTTTCTAGCT 4500
GAAGGAACCT TAAGATCACA TCATCTACTC CTCTACTCCA AATTTCTCAT TCTTCAGGCC 4560
AGGAAACCGA GACACAGAGG TAAAGTAATT TCCCCAAGGT CACACAGCTG GCTGGGGCAG 4620
GATTGGGTTT ACAACCCACA TCTCCTGGCT CTTATTCCAG GGCCTTTTCC CACTAAGTAG 4680
TATTGCCTTC CATTAGGCTC CTGAGAGTTA TTTCTCAGGG TCATGTTGCA TCTTGGAGCC 4740
ACATGCTGCT GCCCTGATCT CAGTGGGAAA TNCACCCAGC AACCTAATAC AGCCCCTTTT 4800
CCCTGCATTC ACCTGGTTCC CATCCACATG GGTTGCAGAT GTCCTTGAAG AGAGTGAGGC 4860
ATTGAGGGCC AATAGGAGCA ATGGGGTCCC TGGCCTTGTC CATCTGATTC AGGAGATCAC 4920
TGCTCCATCG TGAGGAGCCC TCTGAATAGC CCCCCACTGA ATGCTTGCCT TGCCCAAATG 4980
GAATGGAGGA AGATTGATTT TCTCCATCAG TTCACCTTGT GTCATCTCAT AATGGTTGGT 5040
CTTTCCAGGC TGAGGGAAAT GTTTCTTGTT TCCANAGTAN AAAAAAGAAA GAGTGGAACA 5100
ATANCTTTGT TCATCCTAAC TTTCTGAGAT GGCTTTTCAA CATTTAAAAA AAACTAGTGT 5160
GGTACCATTC ACTGGCANGA TTTNTTTTAG AATATGGGAG TAAGATGAGG TAGAGAAAAT 5220
AACCTGGTCT CACTGTGGTT GCCCTCATCC ACAATGTCCC CAAAGCCATC CTGCTNTGAT 5280

GAGGACAATT TCCAGGTATA AGCAAGGGGC TTTGTGACAA AAATGTACCC TGGCTGATGT 5340
TAAACATTGG CTCCTGTGTT TGCACCAAAA TAGCAAGCTG TGTGCTCTAT ACACTCTTCC 5400
CATCGTCTTG TGTACACTGC TCCTGTGGCC TTCCACAGCA GAAACCAGGG CAAAAGGGTC 5460
CAAACACATG GTTTTCCTTG CTGCAAGGCT NTTCCTGGGA ACTAAGGGGG TATTTATTAG 5520
TTCAGTTNTA AGAGACCTCC TTCTGGGCTT ACCCCACTCC TCAGGTACTT CTCTCTCCTT 5580
CCTCCTTCTC CTCCACAGTC ACAAGTAACC AAGGAACCTG AAAGTGGATG TGTAGCTATT 5640
TGAAGAAGGC AAGGAACCCT GAGATTCTTC TTTGAATCCT TTAGTCCAAG TCTTAGACCA 5700
GTGATTGGTG CTTACCTTGA ACAAAATTTT GTCTGTGTTC CTAATCCCTT CAATACTNTG 5760
GGTACAATGC TCCCAATCAC CCTGCACATT TGATTCTAAA TGGCTTTTAT TTTTTAAAAA 5820
TCCATATCCC TAGGACAAGA NAACAGGATG CCTATATCCC CAAAATGAGC TCCAGGACAC 5880
TGATGGGAAT GATCCCAANG ATCACCCCAC CTCAGAAAAC GTCTGTGCCA ANAGACTTCC 5940
CCAGATAGAA NCACTGGGAC AGTGGTTTGA ACGACTTCTT TTATGGTTGT CCAGTTTGCT 6000
ATGGAAATAA AAGGCATTGA TTTTTTAAAA AAGATGATTG GAACCTGTCT TTGGCCACAT 6060
AGGGCCACTT GGATCCATTT CCAGGCCTTA CTCATATATT GCCTTCACTG AAGGGCTTTG 6120
GCTTTAAGTC CCAGACTGGT CTCCCAAGTG AACCATAAGT GTTTTGGAGC TCATCTGGGG 6180
TGAGGCATGA GAATGTTGCC CCATCTATCC CTTCAGGAAA AGGTGCCTTC CCTCCCTTTC 6240
TCCTAAAGCC TGGTCCCCAA AAATTGTTTT TGTCTCCAAA AGTCTAGTAT GGTCTTTATA 6300
CACCCANACT CTTAGTGTTG CGTCCTGCCT TGTTTCCTTG TTAAGGATCT ATGCANACCT 6360
CCCGCTTTGG CTTAGCTAGC GTGACATTGG CTATCATTTG ACAAGACTAA CTTTTTTTTT 6420
TTTTTTTTTG ACTGAGTCTC CCTCTGTCAC CTAGGCTGGA GTGCAGTGGC ACAATCTTGG 6480
CTCGCTGCAA CCTTCACCCT TCACCTCCCA GGTCGAAGCG ATTCTCCTGC CTCAGTCTCC 6540
CGAGTAGCTG GGATTACAGG CGTGCGCCAC CAAATCTGGC TATTTTTTTA TTATTATTAT 6600
TTTTAGTAGA GATGGGGTTT CACCATGTTG GCCAGACTGG TCTTGAACTC TTGGCCTCAA 6660
ATTATCTGCC CACCTCGGCC TCCCAAAGTG CTGGGATTAC AGGCATGAGC ACCATGCCCA 6720
GCTGACAAGA CTAATTTTTT ATCCCTTGGT TTATTGGCTT CAACATCTTC TGGAATCAGA 6780
GGTGATTTTT TCTTACCTTG GATGCCTGAG ACTAGGGGAG TATAGAATTC CAATTGGTAA 6840
TTAAGGCATC TTTCTGCTCC TGATCAGAAG GGCAGGTTAG TTGGGAGAGG TCAGATGGCA 6900
CAACAGAAGT CACCTTGTAA GTAAGGCAAA GACTTTGAAG GCATTAGCGT TTCTCATTAC 6960
TTAGGTCAAT AACCTTGAGG GAATCAATGG CTTTTTTGCC GCTCTACCTC TTTGTGTATC 7020
TCTTTGACTT TTCTTTCTCT GTCTAGTTTC CTCTGTTCTC AGTTTATATT CTATGTTATC 7080
AGTCTCTCTT TCCACAGTAC AAACATCCAT CCTTTCTCCT GTGCAATTCT GTCTCTCCCT 7140
CTTATTATCT TTATTTGTAC TTTTTCCTTC CTCCCTGTCT AGGCATTGGG CATGTGCCTC 7200
TTCTTAGCCT GTGATTTTGC CTTGGGACTG ATGATAAATT ATTTCCAGAT TCAATCAGCC 7260

CTGGTCCTAC CCCAGTCCAA TCAGAAGTAT GTTGGTGGGG AATCAACCTG ATCCTGGCCC 7320
TTTCTTCTTC TCCATTTTCA TTCGTAATCC CCCTCAGCAG ATCTTTACAA GCAGTTTCCT 7380
TATAGCTCAT GTATCTTTAG GTCTTTGCCT TCCAAGCACT GTACAGAATA CTTTGTGGTT 7440
CCTTTTTAGT CTGACATTTT GTGGAGCAGT GAAGCGTGCT CAGAGACATA ATCAGCTGAA 7500
GAGAAAAAAT CCACCCATGG ATTTATATCA GCTAAATACT AATAATTGAT TTTGTTTGAT 7560
GTGCCCATAA TTTTTAAAGC TGCAATATAA TATAATGAGG GACCACAGGT AATTTCTCCT 7620
GTCATTTGTT TTGGCTGGAT GGGGGTGGGG GAGTAATTGC TTAAAGTTTT ACCATTACAC 7680
ATTAAACTCT CTATAATAAT CTTGTTTGGG GCTTGCTAAC TGTTGAGCTG TTTTAACTAA 7740
ACTGGTAGGC AATCGGAGTT GATTTAAATG AAAAGATAAT TTAACAAATC TATACTATAA 7800
AAAGAGACAT TTGCTTAATT GACATGTATT TTTTCCTTCT GAGTCACCTA AACATTTACT 7860
CTTGACACCA ACTGTTCATG ATACTGAATA GACAGTCCAT ATAAGAGAAA TTAGTGGACC 7920
TAAAGAAGCC AGATTGTAGG TGTTAATTTA TTAAACAGAA TTGCAAAGCC CTTGGAAATG 7980
TCACTGCTTG GCAATACCAT ATGGCATGCC AAAATTTACA ATGACTTTTC TTTATAAGTT 8040
ATCCAAAAGG GATTTGAACA AGTAAGAGGT TATGCCAAAA TGTCTCCAAT GTATGGTCCT 8100
GTAATATATT GCAGCTTGAA GCCAATGATC CCTTATGACT TGTATACAAC TAATGCATGT 8160
TTTATTGAAT TTTGCATTTC CCACGTGTGG TAAGTCTTTA AAATGTTTTT GATCACCTTT 8220
NTGTGCCATT AAACTTGTAC AGAAAATGTT TTTATGGCCA TTTTCAAAGG GAGAAAGTTT 8280
AAAATGGAAA CAGCCCACCC TTTCTGCCCT ATAGCTGTAG TTAGAATTGA GTACCTGTAG 8340
CAAAACAGCT GTAATTGGTG GTTGTAGTGT TAGAGGTGTT AGCTTGCTAG TGACTAGCTT 8400
TGGAGAGTAA ATGCATGGTA TTGTACATCA CATTTCTTAA CTCGTTTTAA CCTCTGAAAA 8460
GAATATATTC TTCTTTGTAG TCCTTCTTCC CACCCCCTTG CCCTCTCCCT CTCCCTGCTC 8520
CCAGTTGTCT TACAGTTGTA AATATCTGAT TTGAGGCCCA ATAACTCTTG CCAAGTAAAG 8580
TCAGCAAACA ACAAACAAAC CAAAATGTGG GGAAAAGGCA TTTCTCAACC ATCTCTCAGC 8640
AGTTATTGAT CATTTCTTAA GGAACAGCAT TGTGATCAAA GACTCAACTT TACGTAAAAA 8700
TCAGTGGTAA ATTGGGGTTG TATTGGCCAT TGATTACATT CAGGATTGAA TAGTTTTCAG 8760
AATCACATGT AATCCAAAGA CAGTAGGTAG TGATGTCCCT TATCCCTGCA GCTGTTTTAA 8820
GATAGAGACC TCAGAAGACT CTGCTTGACC GATGACCAAT AATTATTTGA AAAAAAAAGA 8880
AAAAATGAGA GAAATAAAAC AGATATTTAA GAACTTTAGC CACCTATTTA GAATAGTTAT 8940
AGCCAGAAAA AAAAACAAGG GCATGAGTTC AAATGCATTA CTATCAGTGT CCTAGGCAAT 9000
ACCTAACCTA CTCTGAAATT GTGATTCAAA AGCAGTATTT CAAGAGGCAT TCTCCTTTTT 9060
TGGTTTGCTG ACCCCACTTG GACTGGTAGG TTTGGTGAGG CCCCCATAAA CCAGCTGGAG 9120
CAGACCCTTT TCATCTCCTG TGCCTGTAAC ACCCCTCTTC CCCCACCCCC TCCGCAATTC 9180
AATGAGGGCT TTCTTGGGTC AGAGGACTTC AAGGTTGTCT AGAGAAGTTT GCCATGTGTG 9240

TAAGGTGCTG TGAACTGTGA GTGCTGAAGA TTCGCAGCAT TCAATACCAG GCAGCCAAAG 9300

AGCTGCTCTT GCAATTATTT TGGCTCTCAA GCTCTGTTCT TCATCGCATT CTCATTTCTG 9360

TGTACATTTG CAAGATGTGT GTAATGTCAT TTTCCAAAAA TAAAATTTGA TTTCAAT 9417

(2) INFORMATION FOR SEQ ID NO: 20:

(i) SEQUENCE CHARACTERISTICS:
(A) LENGTH: 1105 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 20:

GTCTCTGAGG TTCCACCAAA ATATGGAACT TGATTTTGGA CACTTTGACG AAAGAGATAA 60

GACATCCAGG AACATGCGAG GCTCCCGGAT GAATGGGTTG CCTAGCCCCA CTCACAGCGC 120

CCACTGTAGC TTCTACCGAA CCAGAACCTT GCAGGCACTG AGTAATGAGA AGAAAGCCAA 180

GAAGGTACGT TTCTACCGCA ATGGGGACCG CTACTTCAAG GGGATTGTGT ACGCTGTGTC 240

CTCTGACCGT TTTCGCAGCT TTGACGCCTT GCTGGCTGAC CTGACGCGAT CTCTGTCTGA 300

CAACATCAAC CTGCCTCAGG GAGTGCGTTA CATTTACACC ATTGATGGAT CCAGGAAGAT 360

CGGAAGCATG GATGAACTGG AGGAAGGGGA AAGCTATGTC TGTTCCTCAG ACAACTTCTT 420

TAAAAAGGTG GAGTACACCA AGAATGTCAA TCCCAACTGG TCTGTCAACG TAAAAACATC 480

TGCCAATATG AAAGCCCCCC AGTCCTTGGC TAGCAGCAAC AGTGCACAGG CCAGGGAGAA 540

CAAGGACTTT GTGCGCCCCA AGCTGGTTAC CATCATCCGC AGTGGGGTGA AGCCTCGGAA 600

GGCTGTGCGT GTGCTTCTGA ACAAGAAGAC AGCCCACTCT TTTGAGCAAG TCCTCACTGA 660

TATCACAGAA GCCATCAAAC TGGAGACCGG GGTTGTCAAA AAACTCTACA CTCTGGATGG 720

AAAACAGGTA ACTTGTCTCC ATGATTTCTT TGGTGATGAT GATGTGTTTA TTGCCTGTGG 780

TCCTGAAAAA TTTCGCTATG CTCAGGATGA TTTTTCTCTG GATGAAAATG AATGCCGAGT 840

CATGAAGGGA AACCCATCAG CCACAGCTGG CCCAAAGGCA TCCCCAACAC CTCAGAAGAC 900

TTCAGCCAAG AGCCCTGGTC CTATGCGCCG AAGCAAGTCT CCAGCTGACT CAGCAAACGG 960

AACCTCCAGC AGCCAGCTCT CTACCCCCAA GTCTAAGCAG TCTCCCATCT CTACGCCCAC 1020

CAGTCCTGGC AGCCTCCGGA AGCACAAGGA CCTGTACCTG CCTCTGTCCT TGGATGACTC 1080

GGACTCGCTT GGTGATTCCA TGTAA 1105

(2) INFORMATION FOR SEQ ID NO: 21:

(i) SEQUENCE CHARACTERISTICS:
(A) LENGTH: 360 amino acids
(B) TYPE: amino acid

(C) STRANDEDNESS: single
(D) TOPOLOGY: linear

(ii) MOLECULE TYPE: protein

(vi) ORIGINAL SOURCE:
(A) ORGANISM: Homo sapiens

(xi) SEQUENCE DESCRIPTION: SEQ ID NO: 21:

(2) INFORMATION FOR SEQ ID NO: 22:

    (i) SEQUENCE CHARACTERISTICS:
        (A) LENGTH: 402 amino acids
        (B) TYPE: amino acid
        (C) STRANDEDNESS: single
        (D) TOPOLOGY: linear

    (ii) MOLECULE TYPE: protein

    (vi) ORIGINAL SOURCE:
        (A) ORGANISM: Homo sapiens


    (xi) SEQUENCE DESCRIPTION: SEQ ID NO: 22:

Met Asp Ser Phe Thr Thr Val Ser His Val Gly Met Cys Glu Glu Thr
1               5                   10                  15

Asp Ala Ser Phe Asn Val Phe Ser Pro Lys Phe Gln Phe Asp Arg Ser
            20                  25                  30

His Cys Gln Ser Leu Arg Phe His Gln Asn Met Glu Leu Asp Phe Gly
        35                  40                  45

His Phe Asp Glu Arg Asp Lys Thr Ser Arg Asn Met Arg Gly Ser Arg
    50                  55                  60

Met Asn Gly Leu Pro Ser Pro Thr His Ser Ala His Cys Ser Phe Tyr
65                  70                  75                  80

Arg Thr Arg Thr Leu Gln Ala Leu Ser Asn Glu Lys Lys Ala Lys Lys
                85                  90                  95

Val Arg Phe Tyr Arg Asn Gly Asp Arg Tyr Phe Lys Gly Ile Val Tyr
            100                 105                 110

Ala Val Ser Ser Asp Arg Phe Arg Ser Phe Asp Ala Leu Leu Ala Asp
        115                 120                 125

Leu Thr Arg Ser Leu Ser Asp Asn Ile Asn Leu Pro Gln Gly Val Arg
    130                 135                 140

Tyr Ile Tyr Thr Ile Asp Gly Ser Arg Lys Ile Gly Ser Met Asp Glu
145                 150                 155                 160

Leu Glu Glu Gly Glu Ser Tyr Val Cys Ser Ser Asp Asn Phe Phe Lys
                165                 170                 175

Lys Val Glu Tyr Thr Lys Asn Val Asn Pro Asn Trp Ser Val Asn Val
            180                 185                 190

Lys Thr Ser Ala Asn Met Lys Ala Pro Gln Ser Leu Ala Ser Ser Asn
        195                 200                 205

Ser Ala Gln Ala Arg Glu Asn Lys Asp Phe Val Arg Pro Lys Leu Val
    210                 215                 220

Thr Ile Ile Arg Ser Gly Val Lys Pro Arg Lys Ala Val Arg Val Leu
225                 230                 235                 240

Leu Asn Lys Lys Thr Ala His Ser Phe Glu Gln Val Leu Thr Asp Ile
                245                 250                 255

Thr Glu Ala Ile Lys Leu Glu Thr Gly Val Val Lys Lys Leu Tyr Thr
            260                 265                 270

Leu Asp Gly Lys Gln Val Thr Cys Leu His Asp Phe Phe Gly Asp Asp
        275                 280                 285

Asp Val Phe Ile Ala Cys Gly Pro Glu Lys Phe Arg Tyr Ala Gln Asp
    290                 295                 300

Asp Phe Ser Leu Asp Glu Asn Glu Cys Arg Val Met Lys Gly Asn Pro
305                 310                 315                 320

Ser Ala Thr Ala Gly Pro Lys Ala Ser Pro Thr Pro Gln Lys Thr Ser
                325                 330                 335

Ala Lys Ser Pro Gly Pro Met Arg Arg Ser Lys Ser Pro Ala Asp Ser
            340                 345                 350

Ala Asn Gly Thr Ser Ser Ser Gln Leu Ser Thr Pro Lys Ser Lys Gln
        355                 360                 365

Ser Pro Ile Ser Thr Pro Thr Ser Pro Gly Ser Leu Arg Lys His Lys
    370                 375                 380

Asp Leu Tyr Leu Pro Leu Ser Leu Asp Asp Ser Asp Ser Leu Gly Asp
385                 390                 395                 400

Ser Met

(2) INFORMATION FOR SEQ ID NO: 23:

    (i) SEQUENCE CHARACTERISTICS:
        (A) LENGTH: 23 base pairs
        (B) TYPE: nucleic acid
        (C) STRANDEDNESS: single
        (D) TOPOLOGY: linear

    (ii) MOLECULE TYPE: cDNA

    (vi) ORIGINAL SOURCE:
        (A) ORGANISM: Homo sapiens

    (xi) SEQUENCE DESCRIPTION: SEQ ID NO: 23:

TTTCTCTCAG CATCTCCACC CAA                                          23

(2) INFORMATION FOR SEQ ID NO: 24:

    (i) SEQUENCE CHARACTERISTICS:
        (A) LENGTH: 20 base pairs
        (B) TYPE: nucleic acid
        (C) STRANDEDNESS: single

(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 24:

CAAAGCCTGC TCTCTCTGTC 20

(2) INFORMATION FOR SEQ ID NO: 25:

(i) SEQUENCE CHARACTERISTICS:
(A) LENGTH: 317 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 25:

ATTTCTCTCA GCATCTCCAC CCAACAAAGC CTGCTCTCTC TGTCCAAAGG AAAATCCCAG 60

GTAGACCTGG AGATGCTAAC CTTGGGTCAT AGCCTGACAA AATTCCCCTC CCTTGAAGTA 120

GCGGTCCCCA TCGTTTTCCA TCCAGAGTGT AGAGAACGTT GGGATTGACA TTCTTGGTGA 180

TGGATAGACA ATGGTACTCA GACAGGAGAA AGACCAACAT TATATTTCTC TCAGCATCTC 240

CACCCAAACC CTTGAAGTAA CGGTCCCCAG TACTAAGAGG CCTTGCGTTC TAATCTGTGA 300

GGATACAGAT GATGCGA 317

(2) INFORMATION FOR SEQ ID NO: 26:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 26:

CTGGAGATGC TAACCTTGGG T 21

(2) INFORMATION FOR SEQ ID NO: 27:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 27:

ATAGCCTGAC AAAATTCCCC T 21

(2) INFORMATION FOR SEQ ID NO: 28:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 28:

CCTTGAAGTA GCGGTCCCCA 20

(2) INFORMATION FOR SEQ ID NO: 29:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 29:

GTTTTCCATC CAGAGTGTAG AG 22

(2) INFORMATION FOR SEQ ID NO: 30:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 30:

GTTGGGATTG ACATTCTTGG TG 22

(2) INFORMATION FOR SEQ ID NO: 31:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 31:

ATGGATAGAC AATGGTACTC AG 22

(2) INFORMATION FOR SEQ ID NO: 32:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 32:

ACAGGAGAAA GACCAACATT AT 22

(2) INFORMATION FOR SEQ ID NO: 33:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 33:

CCTTGAAGTA ACGGTCCCCA 20

(2) INFORMATION FOR SEQ ID NO: 34:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 34:

CTGGAGATGC TAACCTTGGG TA 22

(2) INFORMATION FOR SEQ ID NO: 35:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 35:

GGTAGCTCCT TCTGTTCTCT T 21

(2) INFORMATION FOR SEQ ID NO: 36:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 36:

AGCCCCACTC ACAGCGCCCA C 21

(2) INFORMATION FOR SEQ ID NO: 37:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 37:

AGGCACTGAG TAATGAGAAG AA 22

(2) INFORMATION FOR SEQ ID NO: 38:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 38:

CAATCCCAAC TGGTCTGTCA AC 22

(2) INFORMATION FOR SEQ ID NO: 39:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 39:

TCTGCCAATA TGAAAGCCCC C 21

(2) INFORMATION FOR SEQ ID NO: 40:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 40:

GATTTCTTTG GTGATGATGA TG 22

(2) INFORMATION FOR SEQ ID NO: 41:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 41:

GTTTATTGCC TGTGGTCCTG AA 22

(2) INFORMATION FOR SEQ ID NO: 42:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 42:

GTTTATTGCC TGTGGTCCTG AA 22

(2) INFORMATION FOR SEQ ID NO: 43:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 43:

CCTTGAAGTA GCGGTCCCC 19

(2) INFORMATION FOR SEQ ID NO: 44:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 44:

GCTTCACCCC ACTGCGGAT 19

(2) INFORMATION FOR SEQ ID NO: 45:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 45:

CTTGTTCTCC CTGGCCTGT 19

(2) INFORMATION FOR SEQ ID NO: 46:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 46:

GCATAGGACC AGGGCTCTTG 20

(2) INFORMATION FOR SEQ ID NO: 47:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 47:

GTTTCCCTTC ATGACTCGGC 20

(2) INFORMATION FOR SEQ ID NO: 48:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 48:

TGAAAGCACC AATAGCCCTG TT 22

(2) INFORMATION FOR SEQ ID NO: 49:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 49:

GGATTTGTAC TCTGGACTCT GA 22

(2) INFORMATION FOR SEQ ID NO: 50 :

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO 50 :

TCCCTTCTTT TTTCCCTTCT CC 22

(2) INFORMATION FOR SEQ ID NO 51 :

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO 51:

TGAGGCAGGT TGATGTTGTC 20

(2) INFORMATION FOR SEQ ID NO 52 :

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO 52 :

ATCCAGGAAC ATGCGAGGCT 20

(2) INFORMATION FOR SEQ ID NO 53 :

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO 53 :

TGACCTGACG CGATCTCTGT 20

(2) INFORMATION FOR SEQ ID NO 54 :

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 54:

ACCTCCCACC AACGGCCACC 20

(2) INFORMATION FOR SEQ ID NO: 55 :

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO 55 :

CCTAATCACT TATTTCTTGC 20

(2) INFORMATION FOR SEQ ID NO 56 :

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO 56 :

TTGGCTAGCA GCAACAGTGC 20

(2) INFORMATION FOR SEQ ID NO 57 :

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO 57 :

AGTTTGATGG CTTCTGTGAT 20

(2) INFORMATION FOR SEQ ID NO 58 :

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO 58 :

GTCCTCACTG ATATCACAGA 20

(2) INFORMATION FOR SEQ ID NO 59:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO 59 :

GTCAACGGAT CATCTAAGAA 20

(2) INFORMATION FOR SEQ ID NO 60 :

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO 60 :

TCACAGGACC ATCATATACA 20

(2) INFORMATION FOR SEQ ID NO 61 :

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO 61 :

ACCCATGGAA ATCCTAAAGG 20

(2) INFORMATION FOR SEQ ID NO 62 :

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO 62 :

CCTCTACTAA GCTGTCTGTG 20

(2) INFORMATION FOR SEQ ID NO 63 :

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO 63 :

TTGTCCTCCA TAAATGAAGT CAG 23

(2) INFORMATION FOR SEQ ID NO: 64 :

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO: 64 :

TTTATCCCTT CCTTTTCTCT 20

(2) INFORMATION FOR SEQ ID NO: 65 :

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO 65 :

AAGAGGTTTA GTAAGGTATA 20

(2) INFORMATION FOR SEQ ID NO: 66 :

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Homo sapiens

(xi) SEQUENCE DESCRIPTION: SEQ ID NO 66 :

AACTTTGTCT CTTCTCTTCT 20

## Description

The present invention relates to the identification of a new gene, called *XLIS,* and of the *XLIS* gene product, called doublecortin, as well as to the diagnostic and therapeutic applications of these nucleotide and peptide sequences.

Development of the six-layered neocortex depends on precisely orchestrated proliferative, migratory, and maturational events (Allendoerfer and Shatz, 1994; McConnell, 1995). During embryogenesis, neocortical cells arise from a proliferative neuroepithelium, the ventricular zone (VZ) adjacent to the lateral ventricle, then waves of cells migrate long distances through changing environments to reach their final destination and settle in an inside to outside order within the developing cortex (reviewed in Rakic, 1988). Investigations of the pattern of cortical cell dispersion suggest that radial migration along radial glia (Rakic, 1972; Luskin et al., 1988) and tangential migration are both involved in cortical specification (Walsh and Cepko, 1992; Tan and Breen, 1993; O'Rourke et al., 1995). Intrinsic to neurons migratory process are decisions about the initiation of migration, the path to be taken, locomotion itself and final position in the appropriate cortex layer. Although none of these processes are understood in detail, there is now cumulative evidence that several classes of molecules, including adhesion molecules, ion channels/receptors, and intracellular cytoskeletal proteins, may all be involved (reviewed in Hynes and Lander, 1992; Rakic and Caviness, 1995; Huttenlocher et al., 1995).

Although once thought to be rare, malformations of the cerebral cortex are increasingly implicated as a major cause of recurrent seizures in children and adults.

Defects in neuronal migration are believed to be implicated in a large heterogeneous group of genetic disorders associated with cortical dysgenesis or gray matter heterotopia (Raymond et al.,1995). These cortical malformations, revealed mainly by the recent widespread clinical use of magnetic resonance imaging (MRI), are increasingly implicated as a major cause of intractable epilepsy and cognitive impairment (Kuzniecky, 1993; Harding, 1996).

Among these cortical dysgenesis conditions, two major distinct malformations of genetic origin have been described: lissencephaly (LIS) or agyria-pachygyria, and subcortical laminar heterotopia (SCLH) or band heterotopia, also referred to as "double cortex" syndrome. SCLH consists of bilateral plates or bands of gray matter located beneath the cortex and ventricle but well separated from both, hence the descriptive term, double cortex. True cortex appears normal in lamination while neurons within the band are scattered with apical dentrites oriented either toward the cortex or inverted (Harding, 1996). Clinical manifestations are mainly epilepsy and mental retardation (Palmini et al., 1991). Skewed sex ratio towards females (51 out of 54 patients) among sporadic patients with SCLH (Dobyns et al, 1996), suggests the involvement of X-linked mutations. Lissencephaly denotes an absence of gyri (agyria) or a reduced number of broadened gyri (pachygyria) and an abnormal thick cortex. The main clinical features associated with lissencephaly are profound mental retardation, intractable epilepsy, feeding problems and shortened lifespan (Aicardi 1991). The most characteristic histological appearance is an absence of the clear neuronal lamination of normal six layered cortex. Instead, it can be roughly demarcated into four-layered cortex overlying a thin periventricular rim of white matter in which are numerous grey heterotopias. The deep abnormal thick neuronal layer which may break up into bands or cells descending into the white matter, suggests an arrest of neuronal migration (Harding, 1996; Houdou et al., 1990; Ross et al., 1997). SCLH and lissencephaly can be observed as sporadic cases or inherited together in a single pedigree. Several families have been recognized in which affected hemizygous males have lissencephaly and heterozygous females have SCLH, suggesting the involvement of an X-linked gene (Pinard et al., 1994; Dobyns et al., 1996).

These inherited malformations provide a unique opportunity to identify genes that orchestrate appropriate neuronal movement to the cerebral cortex and further understand the pathogenesis of this important class of neurological disorders.

Recent genetic mapping studies (des Portes et al, 1997; Ross et al., 1997) localized the gene responsible for X-SCLH/LIS syndrome in Xq22.3-q23. This region was further defined by physical mapping of an (X; autosome) translocation in a girl with lissencephaly (Ross et al., 1997).

The authors of the present invention have now cloned the gene responsible for X-SCLH/LIS syndrome, which they have called *XLIS* gene. They have more precisely isolated and characterized various transcripts resulting from an alternative splicing. Alternative splicing events and potential alternative start sites of transcription are involved in the diversity of transcripts produced by this gene.

Said transcripts contain an open-reading frame (ORF) which encodes a protein of 360 amino acids. Sequence analysis of the cDNA clones corresponding to the 5' end of the transcripts showed three divergent types of sequences : cDNA 1A, cDNA 1B and cDNA 1C. The sequence of cDNA 1C showed an additional ORF encoding for 42 amino-acids which are in frame with the downstream ATG. In order to define the genomic structure of this gene, the authors of the present invention constructed and investigated a cosmid/phage contig that covers the gene. Determination of exon-intron boundaries was performed through sequence comparison between cDNA clones and genomic DNA, which led to the identification of 7 exons. The common ORF is encoded by exon 2 to exon 6 and the initial 54 nucleotides of the last exon. The structure of this gene is unusual in that only 16% of its sequence is coding.

A subject of the present invention is thus an isolated nucleic acid sequence selected from the group consisting of SEQ ID n° 1 to SEQ ID n° 9, a derivative nucleic acid sequence thereof and a homologous nucleic acid sequence thereof.
SEQ ID n° 1 represents the fragment of the genomic DNA of the *XLIS* gene including exon 1A and exon 1B.
SEQ ID n° 2 represents the fragment of the genomic DNA of the *XLIS* Gene including exon 1C and exon 2.
SEQ ID n° 3 represents the fragment of the genomic DNA of the *XLIS* Gene including exon 3.
SEQ ID n° 4 represents the fragment of the genomic DNA of the *XLIS* Gene including exon 4.
SEQ ID n° 5 represents the fragment of the genomic DNA of the *XLIS* Gene including exon 5.
SEQ ID n° 6 represents the fragment of the genomic DNA of the *XLIS* Gene including intron cos 4.
SEQ ID n° 7 represents the fragment of the genomic DNA of the *XLIS* Gene including exon 6.
SEQ ID n° 8 represents the fragment of the genomic DNA of the *XLIS* Gene including intron sc 10.
SEQ ID n° 9 represents the fragment of the genomic DNA of the *XLIS* Gene including exon 7.

A subject of the present invention is also an isolated nucleic acid sequence selected from the group consisting of SEQ ID n° 10 to SEQ ID n° 19, a derivative sequence thereof and a homologous sequence thereof.
SEQ ID n° 10 represents the cDNA fragment corresponding to exon 1A.
SEQ ID n° 11 represents the cDNA fragment corresponding to exon 1B.
SEQ ID n° 12 represents the cDNA fragment corresponding to exon 1C.
SEQ ID n° 13 represents the cDNA fragment corresponding to exon 2.
SEQ ID n° 14 represents the cDNA fragment corresponding to exon 3.
SEQ ID n° 15 represents the cDNA fragment corresponding to exon 4.
SEQ ID n° 16 represents the cDNA fragment corresponding to exon 5.
SEQ ID n° 17 represents the cDNA fragment corresponding to exon 6.
SEQ ID n° 18 represents the cDNA fragment corresponding to exon 7.
SEQ ID n° 19 represents the cDNA fragment corresponding to exon 1C to exon 7.
SEQ ID n° 20 represents the cDNA fragment corresponding to the common open-reading frame (ORF).

"A derivative nucleic acid sequence" is understood as meaning a sequence which differs from the sequences to which it refers by mutation, insertion, deletion or substitution of one or more bases, or by the degeneracy of the genetic code so long as it codes for a polypeptide which is substantially the same as doublecortin.

"A homologous nucleic acid sequence" is understood as meaning a sequence which hybridizes with the sequences to which it refers or to their complementary sequences under the usual conditions of stringency (Sambrook et al, 1989) so long as said homologous sequence shows at least 70 % of homology, preferably 90 % of homology with the above-defined sequences. Said homologous sequences include mammalian genes coding for doublecortin.

The nucleic acid sequences of the invention are useful for the detection of an abnormality, such as a mutation, in the *XLIS* gene or in the transcripts of the *XLIS* gene. Such an analysis allows *in vitro* diagnosis of a neurological disorder associated with said abnormality.

A subject of the present invention is a method of *in vitro* diagnosis of a neurological disorder associated with an abnormality in the *XLIS* gene or in the transcripts of the *XLIS* gene, wherein one or more mutation(s), preferably inducing a modification of the expression of the *XLIS* gene is detected in the *XLIS* gene or in the transcripts of the *XLIS* gene.

The authors of the present invention have more particularly investigated the abnormalities in the *XLIS* gene or in the transcripts of the *XLIS* gene which are responsible for LIS and/or SCLH.

The following table reports a non exhaustive spectrum of null and missense mutations in the *XLIS* gene :

**Table 1**

| The cumulative spectrum of null and missense mutations in the *XLIS* gene | | | | |
|---|---|---|---|---|
| **Mutation type** | **position in cDNA******* | | **Mutation** | **Restriction site Effect of mutation** |
| nonsense | exon 2 (530) | **C**GA → **T**GA | none | R (39) X |
| | exon 5 (1322) | **C**GA → **T**GA | none | R (303) X |
| aberrant splicing(-2 from 780) | exon 3 | **A**G → **G**G | Sty I | splice exon 3 |
| | acceptor site | | premature stop | |
| | exon 4 | **G**T → **A**T | none | splice exon 4 |
| | (+1 from 1223) | donnor site | | premature stop |
| missense | exon 2 (599) | **G**AC → **A**AC | Ava II | D (62) N |
| exon 3 (989) | **C**GG → **T**GG | Sty I | R (192)W | |
| exon 3 (788) | **T**AT → **C**AT | Alu I | Y (125) H | |
| exon 3 (788) | **T**AT → **G**AT | Alu I | Y (125) D | |
| exon 3 (971) | **C**GC → **T**GC | Pst I | R (186) C | |
| exon 3 (971) | **C**GC → **T**GC | Pst I | R (186) C | |
| exon 4 (1164) | A**T**T → A**C**T | None | I (250) T | |

| | | | | |
|---|---|---|---|---|
| * nt position in the cDNA sequence starting from the 5' end of exon 1C (shown on figure 8). | | | | |

Another subject of the present invention is thus an isolated nucleic acid sequence, which differs from the sequences of the invention as above defined, that is to say from the isolated nucleic acid sequences. SEQ ID n° 1 to SEQ ID n° 20, or a derivative nucleic acid sequence thereof or a homologous nucleic acid sequence thereof, by one or more mutation(s) selected from the mutations as defined in table 1.

The present invention relates to methods of *in vitro* diagnosis wherein the nucleic acid sequences of the invention or probes or primers derived thereof are used to detect aberrant synthesis or genetic abnormalities such as genetic rearrangement at the *XLIS* gene level.

The present invention is more particularly directed to a method of *in vitro* diagnosis according to any of claims 14 or 15 comprising the steps of :
- contacting a biological sample containing DNA with specific oligonucleotides permitting the amplification of all or part of the *XLIS* gene, the DNA contained in the sample having being rendered accessible, where appropriate, to hybridization, and under conditions permitting a hybridization of the primers with the DNA contained in the biological sample ;
- amplifying said DNA ;
- detecting the amplification products ;
- comparing the amplified products as obtained to the amplified products obtained with a normal control biological sample, and thereby detecting a possible abnormality in the *XLIS* gene.

The method of the invention can also be applied to the detection of an abnormality in the transcript of the *XLIS* gene, by amplifying the mRNAs contained in a biological sample, for example by RT-PCR.

So another subject of the present invention is a method of *in vitro* diagnosis, as previously defined comprising the steps of :
- producing cDNA from mRNA contained in a biological sample ;
- contacting said cDNA with specific oligonucleotides permitting the amplification of all or part of the transcript of the *XLIS* gene, under conditions permitting a hybridization of the primers with said cDNA ;
- amplifying said cDNA ;
- detecting the amplification products ;
- comparing the amplified products as obtained to the amplified products obtained with a normal control biological sample, and thereby detecting a possible abnormality in the transcript of the *XLIS* gene.

This comparison of the amplified products obtained from the biological sample with the amplified products obtained with a normal biological sample can be carried out for example by specific probe hybridization, by sequencing or by restriction site analysis.

A subject of the present invention is also a nucleic acid sequence which specifically hybridizes with a nucleic acid sequence of the invention as previously defined."

"A sequence which specifically hybridizes [...]" is understood as meaning a sequence which hybridizes with the sequences to which it refers under the conditions of high stringency (Sambrook et al, 1989). These conditions are determined from the melting temperature Tₘ and the high ionic strength. Preferably, the most advantageous sequences are those which hybridize in the temperature range (Tₘ - 5°C) to (Tₘ - 30°C), and more preferably (Tₘ - 5°C) to (Tₘ - 10°C). A ionic strength of 6xSSC is more preferred.

Such sequences, which are useful as primers or probes for the diagnosis methods according to the present invention may be preferably selected from the group consisting of SEQ ID n° 23 to SEQ ID n° 66.
SEQ ID n° 23 represents the oligonucleotide sequence 1AF.
SEQ ID n° 24 represents the oligonucleotide sequence 1BF.
SEQ ID n° 25 represents the oligonucleotide sequence 1BR.
SEQ ID n° 26 represents the oligonucleotide sequence 1CF.
SEQ ID n° 27 represents the oligonucleotide sequence 1CR.
SEQ ID n° 28 represents the oligonucleotide sequence ComR.
SEQ ID n° 29 represents the oligonucleotide sequence Myst2.
SEQ ID n° 30 represents the oligonucleotide sequence ArnAv.
SEQ ID n° 31 represents the oligonucleotide sequence H1.
SEQ ID n° 32 represents the oligonucleotide sequence H4.
SEQ ID n° 33 represents the oligonucleotide sequence CoR.
SEQ ID n° 34 represents the oligonucleotide sequence F1-5.
SEQ ID n° 35 represents the oligonucleotide sequence F1n5.
SEQ ID n° 36 represents the oligonucleotide sequence F2-5.
SEQ ID n° 37 represents the oligonucleotide sequence F2n5.
SEQ ID n° 38 represents the oligonucleotide sequence F3-5.
SEQ ID n° 39 represents the oligonucleotide sequence F3n5.
SEQ ID n° 40 represents the oligonucleotide sequence F4-5.
SEQ ID n° 41 represents the oligonucleotide sequence F4n5.
SEQ ID n° 42 represents the oligonucleotide sequence F1-3.
SEQ ID n° 43 represents the oligonucleotide sequence F1n3.
SEQ ID n° 44 represents the oligonucleotide sequence F2-3.
SEQ ID n° 45 represents the oligonucleotide sequence F2n3.
SEQ ID n° 46 represents the oligonucleotide sequence F3-3.
SEQ ID n° 47 represents the oligonucleotide sequence F3n3.
SEQ ID n° 48 represents the oligonucleotide sequence F4-3.
SEQ ID n° 49 represents the oligonucleotide sequence F4n3.
SEQ ID n° 50 represents the oligonucleotide sequence 2.1 F.
SEQ ID n° 51 represents the oligonucleotide sequence 2.1 R.
SEQ ID n° 52 represents the oligonucleotide sequence 2.2 F.
SEQ ID n° 53 represents the oligonucleotide sequence 2.3 F.
SEQ ID n° 54 represents the oligonucleotide sequence 2.3 R.
SEQ ID n° 55 represents the oligonucleotide sequence 3.1 F.
SEQ ID n° 56 represents the oligonucleotide sequence 3.2 F.
SEQ ID n° 57 represents the oligonucleotide sequence 3.2 R.
SEQ ID n° 58 represents the oligonucleotide sequence 3.3 F.
SEQ ID n° 59 represents the oligonucleotide sequence 3.3 R.
SEQ ID n° 60 represents the oligonucleotide sequence 4 F.
SEQ ID n° 61 represents the oligonucleotide sequence 4 R.
SEQ ID n° 62 represents the oligonucleotide sequence 5 F.
SEQ ID n° 63 represents the oligonucleotide sequence 5 R.
SEQ ID n° 64 represents the oligonucleotide sequence 6 F.
SEQ ID n° 65 represents the oligonucleotide sequence 6 R.
SEQ ID n° 66 represents the oligonucleotide sequence 7 F.

One skilled in the art knows very well the standard methods for analysing the DNA contained in a biological sample and for diagnosing a genetic disorder. Many strategies for genotypic analysis are available (Antonarakis et al., 1989, Cooper et al., 1991).

Preferably, one can use the DGGE method (Denaturing Gradient Gel Electrophoresis), or the SSCP method (Single Strand Conformation Polymorphism) for detecting an abnormality in the *XLIS* gene. Such methods are preferably followed by direct sequencing. The RT-PCR method may be advantageously used for detecting abnormalities in the *XLIS* transcript, as it allows to visualize the consequences of a splicing mutation such as exon skipping or aberrant splicing due to the activation of a cryptic site. This method is preferably followed by direct sequencing as well. The more recently developped technique using DNA chip can also be advantageously implemented for detecting an abnormality in the *XLIS* gene (Bellis et al., 1997).

The cloning of the *XLIS* gene, as well as the identification of various mutations responsible for neurological disorders according to the invention, allow direct or semi-direct diagnosis. The specificity and reliability of such diagnosis methods are more particularly appreciable for prenatal diagnosis. The nucleic acid sequences of the present invention represent a highly interesting tool for genetic counseling.

Defects in the *XLIS* gene, or in the *XLIS* gene product cause syndromes or diseases involving abnormal neurone migration, mainly in the neocortical part of the brain, leading to an abnormal organization of the cortex.

The *XLIS* gene would be more particularly involved in incurable cryptogenic epilepsies and in genetic disorders such as those associated with cortical dysgenesis or gray matter heterotopia (Raymond et al, 1995).

In particular, the inventors have presently shown that defects in *XLIS* gene are responsible for the X-linked lissencephaly and subcortical laminar heterotopia, or double cortex syndrome.

First, *XLIS* gene maps to the potential genetic locus in Xq22 identified by linkage analyses (des Portes al., 1997; Ross et al., 1997). The mapping of the cDNA in Xq22 was ascertained by several hybridizations using cDNA clones as probes on genomic Southern blots containing YACs DNA covering the critical region and DNA from two somatic hybrids containing either the whole human X chromosome or a translocated derivative chromosome that has retained most of the long arm of the X chromosome. Second, *XLIS* gene is expressed in early embryonic brain neurons. Third, missense mutations in *XLIS* gene leading to drastic amino acid changes, and co-segregating with the phenotype, were identified in unrelated families. In each family, the same mutation was identified in hemizygous males affected with lissencephaly and heterozygous females affected with SCLH, confirming the common genetic origin of these two apparently different phenotypes. Fourth, an addition screening for mutations, by denaturing gradient gel electrophoresis (DGGE) and direct sequencing, in sporadic cases of SCLH allowed to identify other mutations including two nonsense mutations (table 1).

The difference in phenotypes between males and females can be explained as follows: In hemizygous males with mutations in the *XLIS* gene, absence of functional doublecortin in all cells of the developing brain will lead to a generalized abnormal organization of the neocortex resulting in lissencephaly or pachygyria. In contrast, in females with SCLH functional doublecortin is absent only in cell populations which inactivate the X chromosome bearing the normal allele, leading therefore to a less severe neocortical dysgenesis. Despite normal cortical histogenesis and cellular connections (Harding, 1996), it appears that heterotopic cells are not rescued by neighbouring cells. This is in line with the putative neuronal intracellular localization of doublecortin.

The hypothesis that SCLH and LIS phenotypes result from a loss of function of the *XLIS* gene is supported by the identification of nonsense and frameshift mutations, and a *de novo* mutation at one of the invariant dinucleotides GT of the 5' donor site resulting in an exon skipping event with a frameshift. This latter mutation was detected in an atypical sporadic case as it concerns a female affected with extended SCLH and pachygyria, and corpus callosum agenesia.

Expression of *XLIS* gene during brain development assessed by Northern blot, *in situ* hybridization and RT-PCR suggests that *XLIS* transcript is present at a very high level in fetal brain and especially in neurons, and is then gradually downregulated and reaches an undetectable level (by Northern blot) in adult brain. This high and diffuse expression of *XLIS* gene in fetal neurons including precursors supports the involvement of doublecortin in the complete disorganization of normal six layered-cortex observed in lissencephaly.

The *XLIS* gene encoding for doublecortin, expressed in fetal neuronal cells including precursors, seems to be required for initial steps of neuronal dispersion and cortex lamination.

Furthermore, as some mutations have been found in atypical cases of SCLH, either a "form fruste" (mother of a family with somatic mosaicism), or severe forms leading to pachygyria and corpus callosum agenesia, mutations in *XLIS* gene are expected to contribute to other cortical dysgeneses. For instance, two pedigrees of X-linked dominant pachygyria in males with decreased expressivity in carrier females previously reported (Berry-Kravis et al., 1994 ; Zollino et al., 1992) may be allelic disorders of the *XLIS* gene. In addition, the involvement of *XLIS* gene in some focal dysgenesis and corpus calosum agenesia is expected. Most certainly, screening of *XLIS* and other related genes like *XLIS-homologous* opens new fields in understanding cortical malformations and child epilepsy.

The open reading frame common to all types of transcript encodes a protein of 360 amino acids named doublecortin. Alternative splicing involving exon 1C, leads to a *XLIS* gene product which is composed of 402 amino acids.

Doublecortin has no significant homology to any protein of known function, except with a gene product of 729 amino acids (GeneBank accession number AB002367, gene called AA0369) reported in a recent large scale study of brain cDNA clones (Nagase et al., 1997), named *XLIS homologous*.

The co-expression of *XLIS* gene product in fetal brain with the *XLIS-homologous* gene may suggest that doublecortin could regulate function of the *XLIS*-homologous protein either via a competitive interaction with upstream and downstream effectors, or a modulation of *XLIS*-homologous kinase activity.

The present invention also relates to an isolated *XLIS* polypeptide substantially having the aminoacid sequence encoded by a nucleic acid sequence of the *XLIS* gene according to the invention , i.e. an isolated *XLIS* polypeptide which is substantially the same as doublecortin

The above expression "substantially" is understood as meaning that said isolated *XLIS* polypeptide exhibits the same biological and immunological properties, as native doublecortin.

More particularly said aminoacid sequence may be selected from the group consisting of SEQ ID n° 21 and SEQ ID n° 22, and a derivative aminoacid sequence thereof.
SEQ ID n° 21 represents the 360 aminoacid protein.
SEQ ID n° 22 represents the 402 aminoacid protein.

"A derivative aminoacid sequence" is understood as meaning a sequence which differs from the sequences to which it refers by mutation, insertion, deletion or substitution of one or more aminoacids, without inducing modification of biological and immunological properties. Said derivative aminoacid sequence shows at least 70% of homology, preferably 90% of homology with the doublecortin polypeptide having the aminoacid sequence as above described.

The "biological properties" of the polypeptides of the invention refer to the activity of doublecortin in the central nervous system (CNS), and more particularly to its activity on the neuronal migration, more particularly in embryonic neocortex.

The "immunological properties" of the polypeptides of the invention refer to the ability of the polypeptides of the invention to induce an immunological response mediated by antibodies which recognize the polypeptides of the invention.

The polypeptides according to the invention can be obtained by any of the standard methods of purification of soluble proteins, by peptide synthesis or by genetic engineering. Said techniques comprise the insertion of a nucleic acid sequence coding for a peptide of the invention into an expression vector, such as a plasmid, and the transformation of host cells with the expression vector, by any of the methods available to the skilled person, like for instance electroporation.

The present invention thus relates to vectors for cloning and/or expression comprising a nucleic acid sequence of the invention and to host cell transfected with these vectors. The expression vector according to the invention comprises a nucleic acid sequence encoding a polypeptide of the invention. Said vector contains a promoter sequence, signals for initiation and termination of translation, as well as appropriate regions for regulation of translation. Its insertion into the host cell may be transient or stable. Said vector may also contain specific signals for secretion of the translated protein.

These various control signals are selected according to the host cell which may be inserted into vectors which self-replicate in the selected host cell, or into vectors which integrate the genome of said host.

Host cells may be prokaryotic or eukaryotic, including but not limiting to bacteria, yeasts, insect cells, mammalian cells, including cell lines which are commercially available.

A subject of the present invention is also a method for producing a recombining *XLIS* polypeptide, wherein said host cell is transfected with said expression vector and is cultured in conditions allowing the expression of a polypeptide according to the invention.

The present invention also relates to monoclonal or polyclonal antibodies, or fragments thereof, or chimeric or immunoconjugate antibobies, which are capable of specifically recognizing a polypeptide according to the invention.

Polyclonal antibodies can be obtained from serum of an animal immunized against doublecortin, which can be produced by genetic engineering for example, as above described, according to standard methods well-known by one skilled in the art.

Monoclonal antibodies can be obtained according to the standard method of hybridoma culture (Kohler and Milstein, 1975).

The antibodies of the present invention can be chimeric antibodies, humanized antibodies, or antigen binding fragments Fab and F(ab')2. They can also be immunoconjugated or labelled antibodies.

Said antibodies are particularly useful for detecting or purifiyng a polypeptide according to the invention in a biological sample.

They are more particularly useful for detecting an abnormal expression of doublecortin in connection with neurological disorders, including not only constitutional genetic disorders but also neurodegenerative disease, such as Alzeiheimer's disease and cognitive impairments related to aging.

Furthermore doublecortin can advantageously be used as a marker of neuronal cells at early stage of development, said marker being easily detected by labeled antibodies of the invention.

Another subject of the present invention is a pharmaceutical composition comprising a purified doublecortin polypeptide of the invention and/or a homologous polypeptide thereof, an isolated nucleic acid sequence encoding said polypeptides, or an anti-sense sequence capable of specifically hybridizing with a nucleic acid sequence encoding said polypeptides, or an antibody directed against said polypeptides, in association with a pharmaceutically acceptable carrier.

Preferably the present invention is directed to a pharmaceutical composition comprising a purified doublecortin polypeptide of the invention and/or a homologous polypeptide thereof, in association with a pharmaceutically acceptable carrier.

The expression "homologous polypeptide", as active ingredient of a pharmaceutical composition, refers to a polypeptide with a homology of at least 40 %, preferably of at least 60 % in comparison to doublecortin. Preferably said homologous polypeptide is for example the protein AA0369 (GeneBank accession number AB002367).

The pharmaceutical compositions of the invention are useful for preventing or treating neurological disorders, wherein doublecortin or the doublecortin *homologous protein* is implicated. The disorders which are more particularly aimed at are disorders of the central nervous system in connection with the axonal development, including cortical dysgenesis or gray matter heterotopia, such as lissencephaly and subcortical laminar heterotopia, as well as cryptogenic epilepsies or neurodegenerative diseases, such as Alzeiheimer's disease.

The pharmaceutical compositions of the invention may be administered to a mammal, preferably to a human, in need of a such treatment, according to a dosage which may vary widely as a function of the age, weight and state of health of the patient, the nature and severity of the complaint and the route of administration.

The appropriate unit forms of administration comprise oral forms such as tablets, gelatin capsules, powders, granules and oral suspensions or solutions, sublingual and buccal administration forms, subcutaneous, intramuscular, intravenous, intranasal or intraoccular administration forms and rectal administration forms.

A further subject of the present invention is a method of preventing and/or treating neurological disorders resulting from defects in the *XLIS* gene or in the *XLIS* gene product, namely doublecortin, in the gene encoding the doublecortin *homologous* protein or in the doublecortin *homologous* protein, which comprises administering to a subject in need of a such treatment an amount of a pharmaceutical composition as above defined effective to prevent and/or alleviate said neurological disorders.

### LEGENDS TO FIGURES :

Figure 1 represents the *XLIS* genomic region with bands Xq22.3-q23.

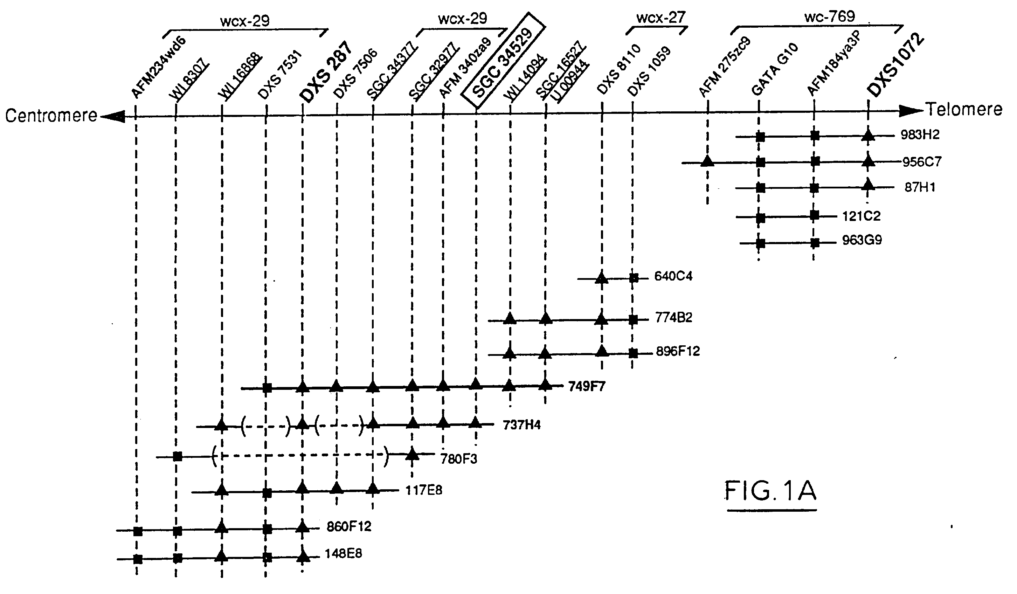

Figure 1a represents a schematic presentation of YAC contig (23 clones), within bands Xq22.3-q23, between polymorphic markers *DXS1210* and *DXS1072* (in bold). Genetic distance between these two markers was about 3.6 cM (Dib et al., 1996). Upper line indicates YAC contigs reported by the Whitehead Institute/MIT Center data base. The order of these contigs is represented according to our data. STSs and ESTs (underlined) used for YAC clones ordering and ESTs mapping, were amplified by PCR on YAC DNA. Some were confirmed by hybridization of HindIII digested YACs DNA blots (triangles). Markers order within contig wc-769 remains unknown. EST *SGC34529*, part of *XLIS* gene, is boxed.
Fig 1b represents a fetal and adult multiple tissue Norther blot hybridized with *SGC34529* probe. A strong and unique tissue specific signal was detected in fetal brain, after 12 hours exposition at -80°C.
Figure 2 represents *XLIS* cDNA contig and schematic presentation of the 9.5 kb consensus transcript.
   Only two ESTs and the minimal set of 8 overlapping cDNA clones (out of 79 positive clones identified) are shown. Five cDNA clones and the EcoRI-HindIII fragment from sc22 genomic subclone were used for successive screenings of fetal brain cDNA library. The number of positive clones is indicated in brackets. In the 5' region, three types of clones (cDNA1A, 1B and 1C) were detected after screening with cDNA 58 ; for each type, the number of identical clones is indicated in square brackets. Open reading frames (ORF) are shown : bold line corresponds to the common ORF, bold dotted line corresponds to the additional in-frame ORF present only in cDNA 1C. The EcoRI (◇) and HindIII (△) restriction sites and the Alu sequence are indicated on the consensus cDNA. Sc22 clone (dotted line) is a HindIII genomic subfragment isolated from a cosmid clone containing the 3' untranslated region of *XLIS* gene.

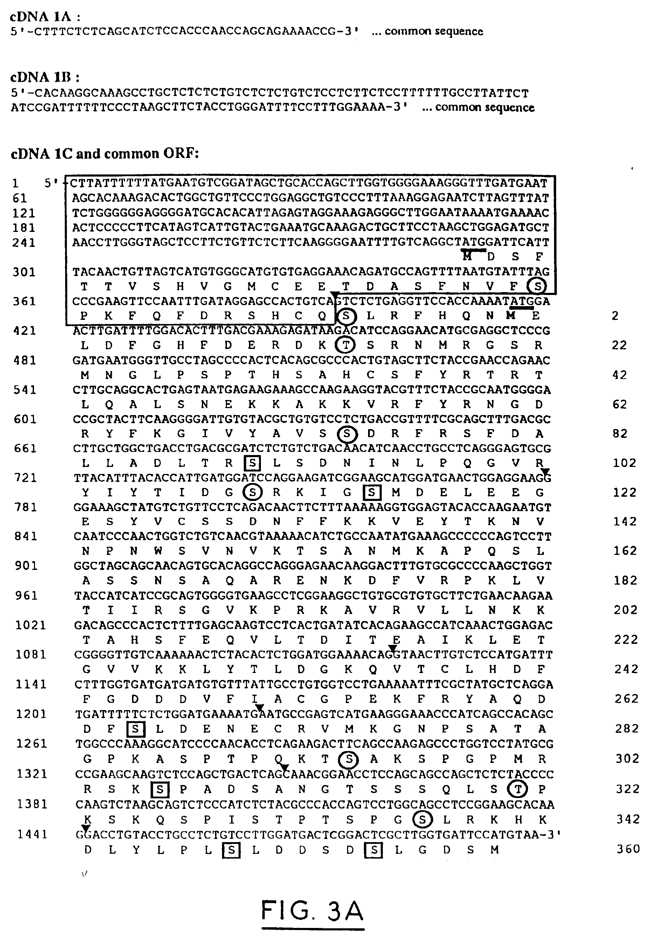

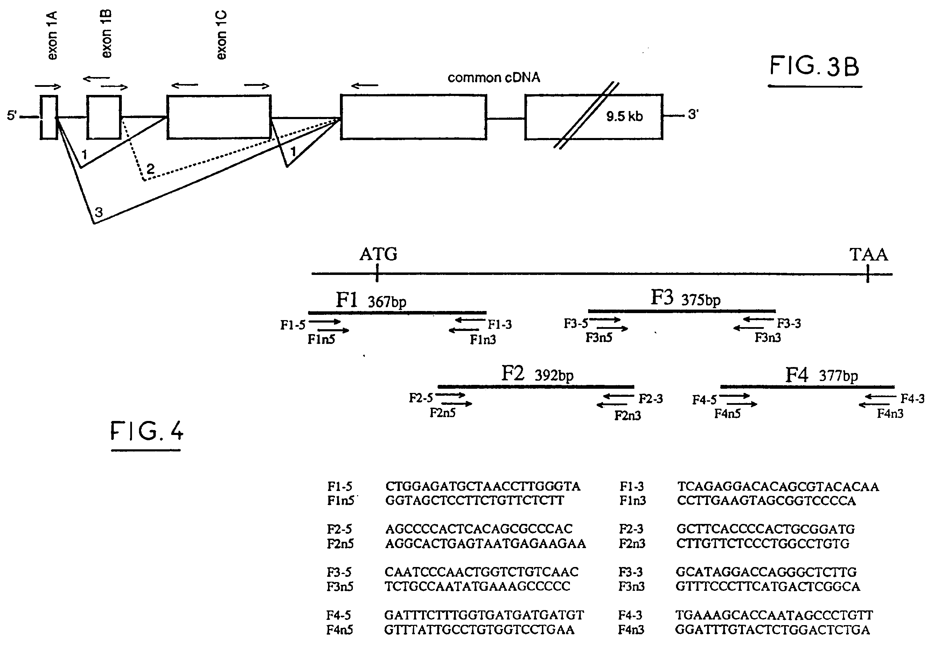

Figure 3 represents primary structures of *XLIS* cDNA and predicted protein.
Figure 3a coding cDNA and deduced amino acide sequences of *XLIS*.
   Nucleotide sequences of exons 1A, 1B, 1C (boxed sequence) and common ORF are shown. ATG codons representing the putative translation start sites are underlined. The predicted common ATG (lower one) with the first in-frame stop codon (TAA), yields an open reading frame of 1080 bp that encodes a predicted protein of 360 amino acids. The upstream in frame ORF, starting at the ATG within exon 1C (boxed sequence), encodes for 42 additional amino acids. Potential PKC and CK2 phosphorylation sites are shown as open circles and squares respectively.
Figure 3b represents genomic structure of the 5'region of *XLIS* gene.
   RT-PCR experiments using total fetal brain RNA were performed with different couples of primers (arrows) located in the three upstream exons 1A, 1B, 1C and the first common exon. Forward (F) primers are oriented to the right and reverse (R) primers are oriented to the left. Sequences of the primers are as follows:
   1AF 5'-TTTCTCTCAGCATCTCCACCCAA-3',
   1BF 5'-CAAAGCCTGCTCTCTCTGTC-3',
   1BR 5'-CAAAGGAAAATCCCAGGTAGA-3',
   1CF 5'-CTGGAGATGCTAACCTTGGGT-3',
   1CR 5'-ATAGCCTGACAAAATTCCCCT-3',
   ComR 5'-CCTTGAAGTAGCGGTCCCCA-3'.
   Identified splicing events, corresponding to 3 different transcript isoforms, are indicated with continuous and dotted lines.
Figure 4 represents primer sequences and sizes of expected nested RT-PCR products.
   External primers were used for the first round of RT-PCR and internal primers for the second round of PCR (experimental procedure). Amplified overlapping fragments cover the coding sequence that starts at the common ATG.

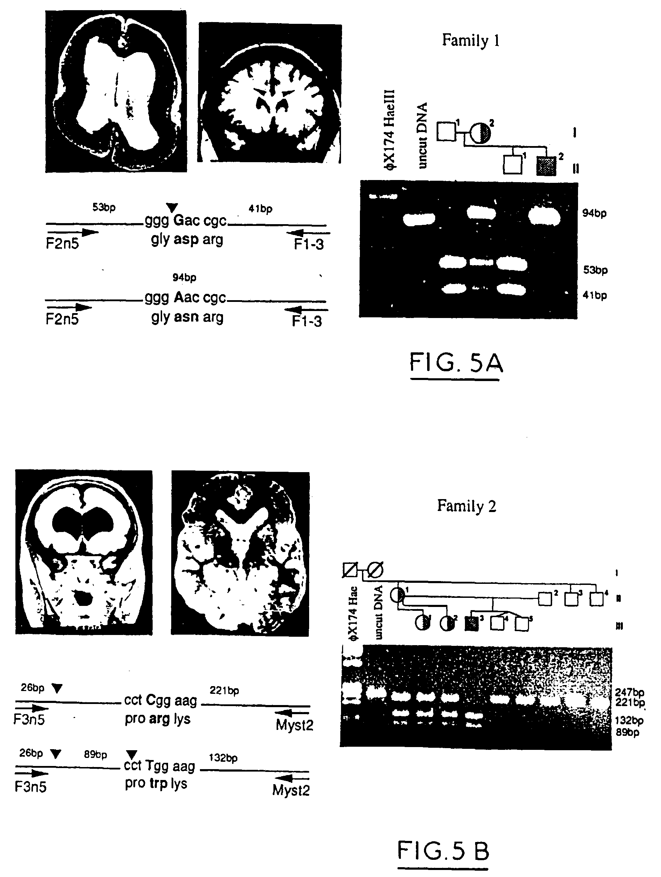

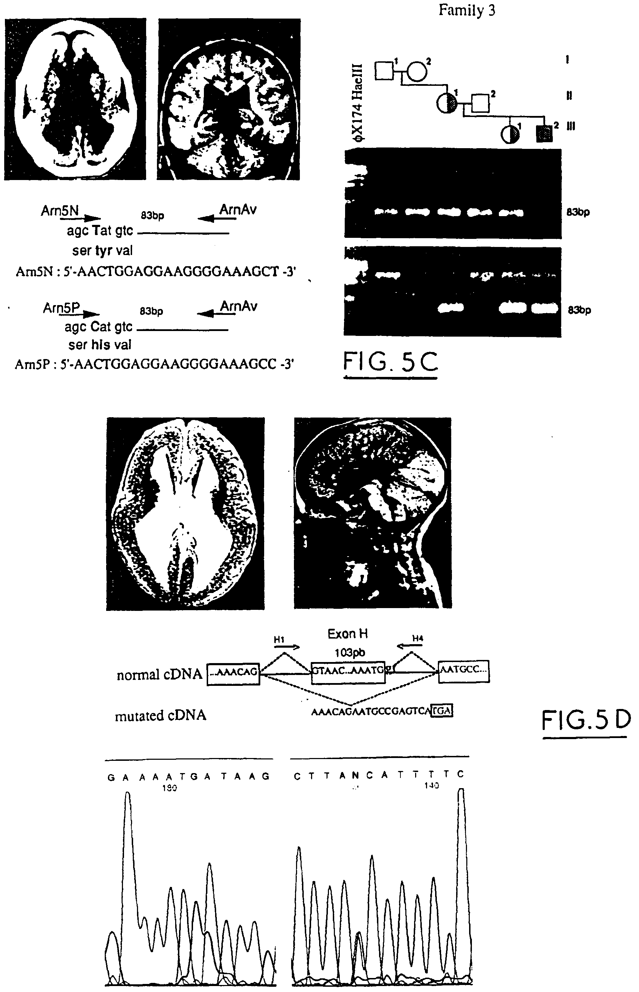

Figure 5 represents identification of mutations in *XLIS* gene and their segregation in X-SCLH/ILIS families.
Figure 5a : family 1
   MRI images of affected mother (I-2) and her son (II-2) show bifrontal SCLH (arrows, right coronal image) and generalised agyria (left axial image), respectively. The G to A mutation was detected in individual II-2 patient. As the mutation disrupts an AvaII site (lower sequence), genomic PCR products using primers F2n5 and F1-3 (previously tested on genomic DNA) were digested and analysed on 2 % Nuesieve gel, for all members of family 1. DNA from the affected son remains uncut, while expected products (53 bp and 41 bp) were obtained for the healthy son and the father. The heterozygous female (I-2) line shows three bands, confirming the presence of both alleles.
Figure 5b : family 2.
   Three affected children are born to the same affected mother but to three different fathers. Axial MRI image of one affected female (III-2) shows extended SCHLH (right) and coronal MRI image of her brother (III-3) shows bifrontal agyria-pachygyria (left). As the C to T mutation creates an Styl restriction site, genomic PCR product (with primers F3n5 and Myst2) from affected son is digested in three fragments (132 bp, 89 bp and 26 pb undetectable on the gel); only two products (221 bp and 26 bp) were obtained for the healthy males. Both alleles are shown for the three heterozygous females. Myst2 sequence is: 5'-GTTTTCCATCCAGAGTGTAGAG-3'.
Figure 5c : family 3.
   Coronal MRI image (right) of affected daughter (III-1) and axial CT scan (left) of affected son (III-2) show extended and thick SCLII and agyria, respectively. The T to C mutation was detected in individual III-2. Allele specific genomic PCR was performed. Using normal forward primer (Arn5N, upper gel), an expected PCR product (83 bp) was obtained for all individuals except the affected boy, while using a 3'-mutted one (Arn5P, lower gel), a specific PCR product was exclusively amplified with DNA of the heterozygous females and the affected male.
   ArnAv sequence: 5'-GTTGGGATTGACATTCTTGGTG-3'.
Figure 5d represents sporadic case (JM) with abnormal skipping of exon H.
   Axial (left) and sagittal (right) MRI images show extended agyriapachygyria and complete corpus callosum agenesia, respectively.
   A shorter nested PCR product of fragment 3 was amplified in this patient. The cDNA sequence exhibited a skipping of exon H (103 bp) which induces a frameshift and a premature stop codon, 5 residues downstream the abnormal splice.
   Lower, electrophoregrams of the genomic DNA sequence of intron-exon H junctions using forward primer H1 (left) and reverse H4 (right) showed an heterozygous G to a mutation at the donor splice site
   (H1 : 5'-ATGGATAGACAATGGTACTCAG-3' ; H4 : 5'-ACAGGAGAAAGACCAACATTAT-3').

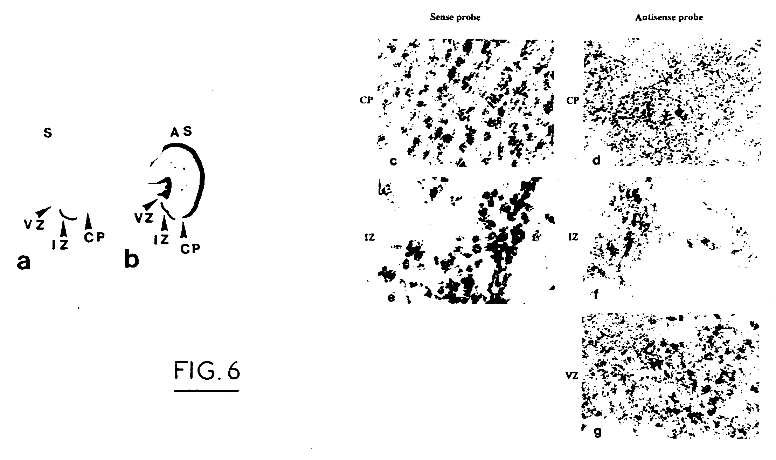

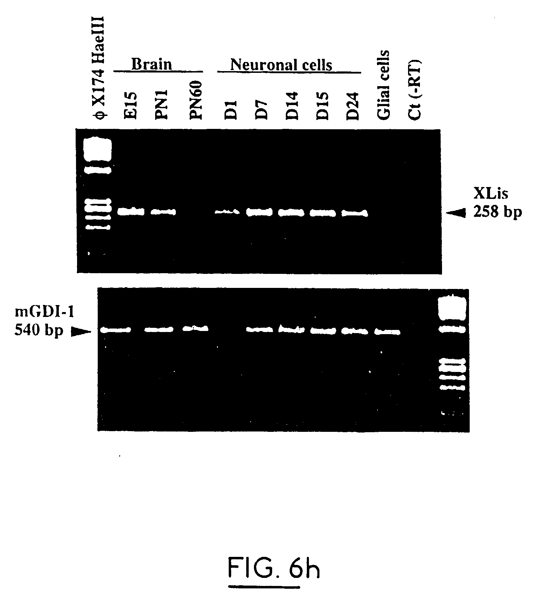

Figure 6 represents *XLIS* gene expression.
Figures 6a to 6g represent *in situ* hybridization analysis of *XLIS* expression.
   32P-labelled sense (S) and antisense (AS) probes were hybridized to coronal sections of human fetal brain (parieto-frontal cortex).
Figures 6a to 6b represent autoradiograms of hybridized sections. Strong signal was observed in the ventricular zone (VZ) and cortical plate (CP), and moderate signal in the intermediate zone (IZ) with the antisense probe, no significant signal was detected with the sense probe.
Figures 6c to 6g represent higher magnification (x 40) of the same sections showing accumulation of silver grains within cells of the CP, IZ and VZ. In the IZ, cells are organized as oriented chains. There was no specific labelling using the sense probe as control (figures c and e).
Figure 6h represents expression study by RT-PCR of *XLIS* gene in mouse brain and in neuronal and glial cultured cells. *mGDI-1* was used as control. The structure of the 5' region of mouse gene is similar to the human one. Nucleotide sequences homology (calculated for 360 coding bases in the common exon) is 89 %, and amino acid sequence identity (for the first 120 residues downstream the common ATG) is 99 %. Mouse 1AF (5'-TTTCTCTCAGCATCTCCACCCAA-3') and mouse CoR (5'-CCTTGAAGTAACGGTCCCCA-3') primers used to amplify the mouse *XLIS* transcript are in the exons equivalent to exon 1A and the first common exon. The amplified fragment results from the alternatively spliced transcript lacking exon 1C. Primers used to amplify the mouse GDI-1 transcript are forward 5'-GAGGCCTTGCGTTCTAATCTG, reverse 5'-TGAGGATACAGATGATGCGA (Shisheva et al., 1994). (E) embryonic, (PN) postnatal, (D) days of culture. Number of days for neurons culture is indicated on the figure.

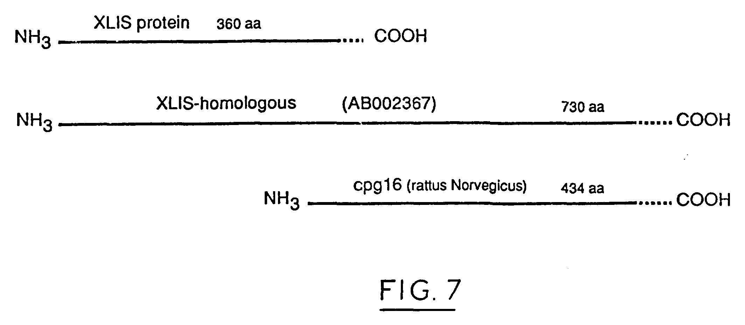

Figure 7 represents amino acid sequence homology of *XLIS* protein with two protein kinases.
   *XLIS* protein shares homology only with the N-terminal part of the Gen Bank *ABOO2367* gene product, also named *XLIS*-homologous. The C-terminal part of the AB002367 gene product and the rattus norvegicus cpg16 (Gen Bank US78857), share significant homology with many calcium calmodulin dependent protein kinases. Dotted lines indicate divergent sequences.

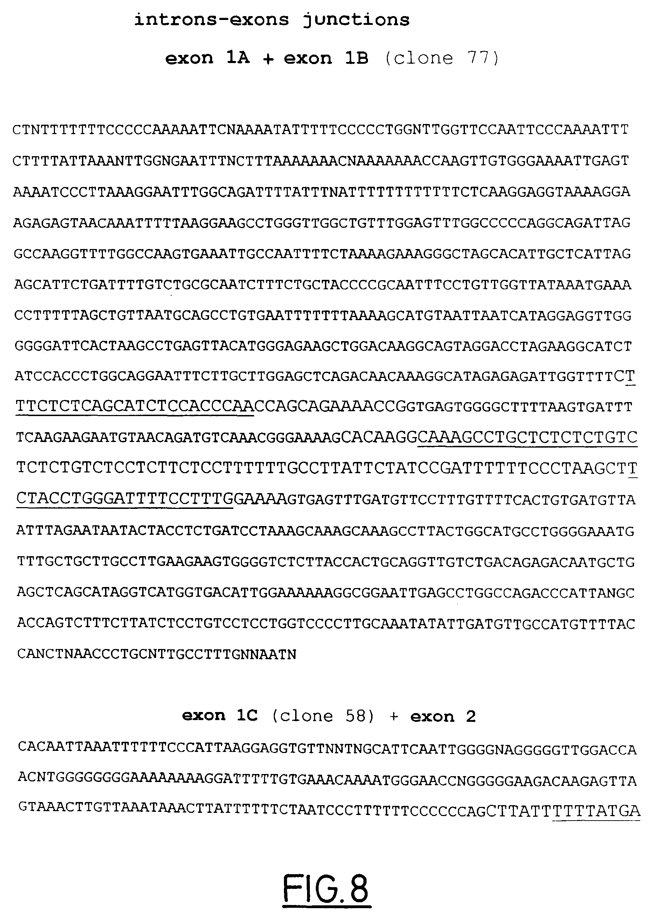

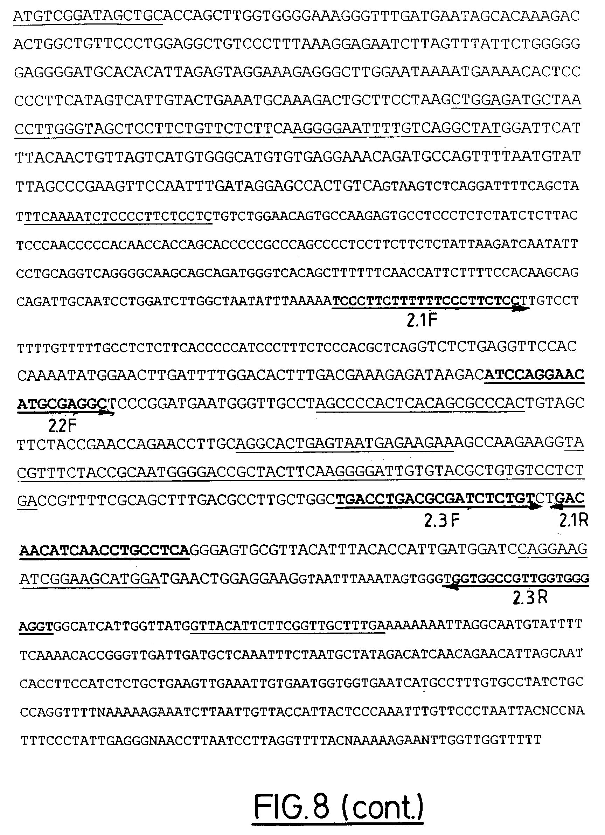

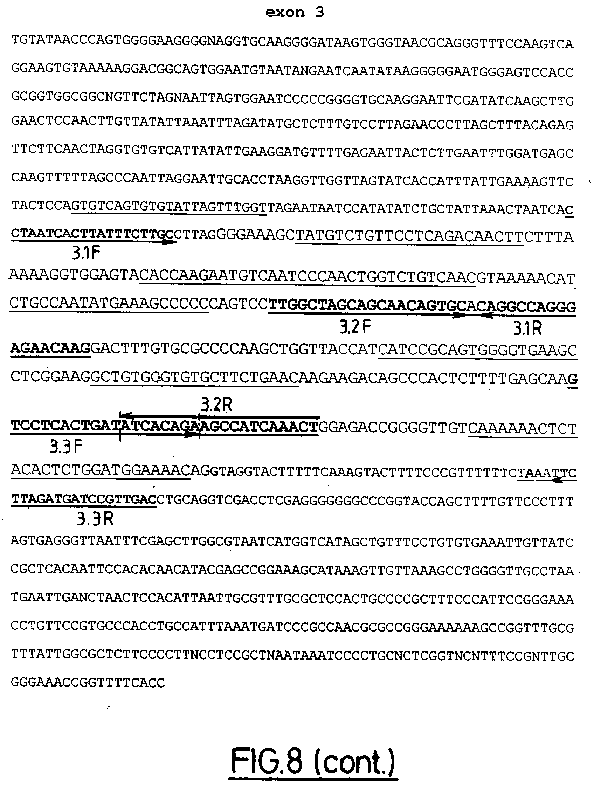

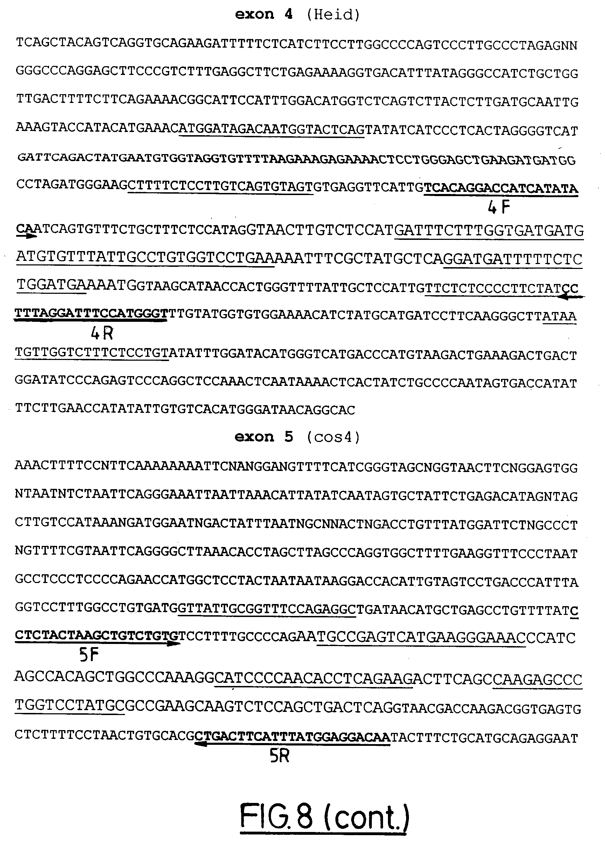

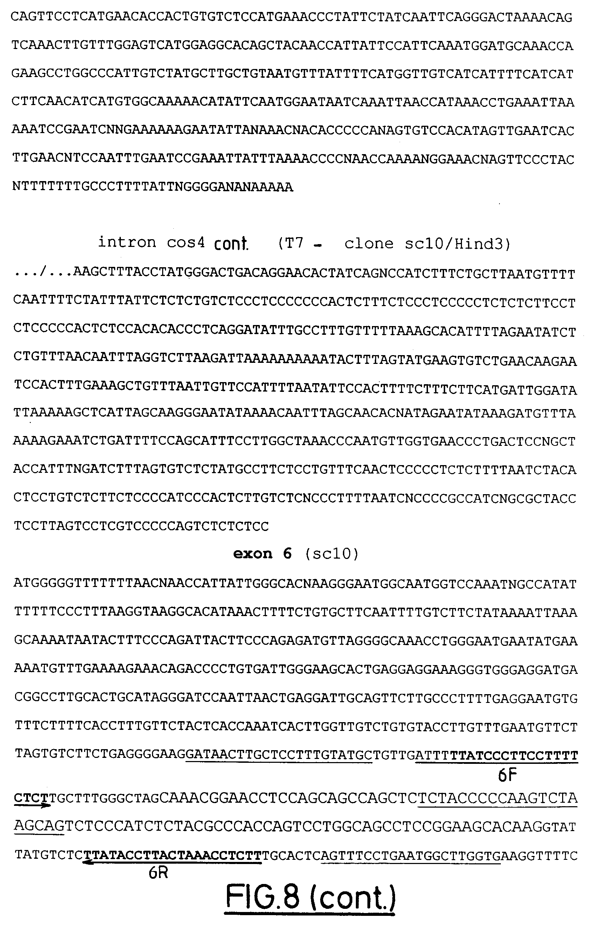

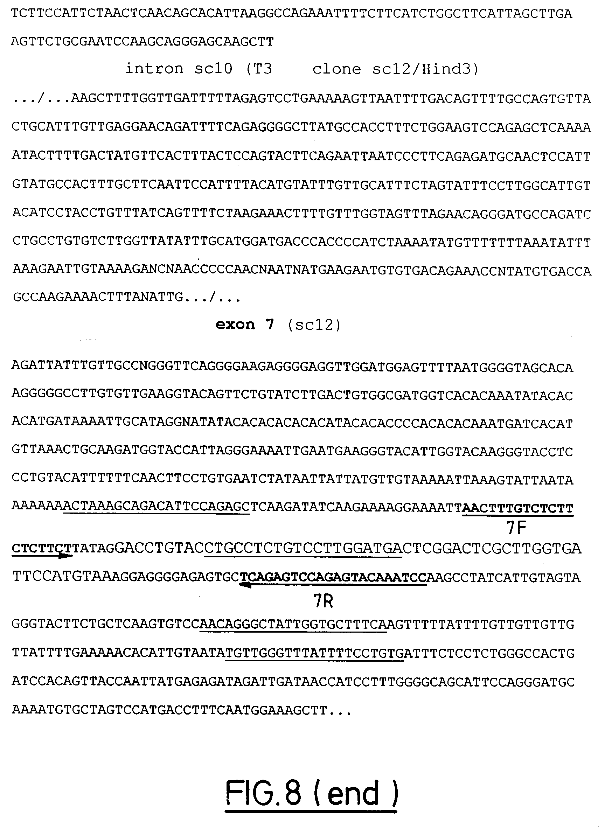

Figure 8 represents fragments of the genomic DNA corresponding to the *XLIS* gene showing the introns/exons junctions. The underlined sequences are oligonucleotides used as probes or primers according to the invention. The capital letters represent the exon sequences and the small letters represent the intron sequences.

### EXAMPLE 1: Identification of the XLIS gene :

### 1. Experimental procedures

### a) Family Material

Clinical data and diagnosis concerning the three X-SCLH/LIS families analysed hereafter were described by des Portes et al., (1997). The sporadic case JM is a 5 years old female born to nonconsanguinous healthy parents (both have normal MRI). She has seizures since 9 months of age, and severe developmental delay with severe cognitive impairment. MRI showed diffuse thick cortex with agyria and pachygyria associated with an extended atypical aspect of SCLH and unexpected complete corpus callosum agenesia (figure 5d). The second sporadic case DO is a 15 years old female born to healthy parents. She has a severe mental deficiency and an intractable epilepsy; MRI shows thick subcortical laminar heterotopia.

### b) YAC clones, STS and EST analysis

YAC clones of *XLIS* critical region were obtained from the UK HGMP Resource Centre. Preliminary YACs ordering data were available on line (CEPH-Généthon and Whitehead Institute/MIT Center data bases). Analysed STSs and ESTs were selected according to the available physical and radiation hybrid maps on the World Wide Web site at http://www.ncbi.nlm.nih.gov/SCIENCE96/. Primer sequences corresponding to these STSs and ESTs were also available in the same Wold Wide Web site. YAC clones were grown in selective media, and DNA was prepared using standard protocols. YAC overlaps and EST mapping were confirmed by a combination of STS/EST amplification and hybridization approaches.

### c) cDNA isolation and characterisation

Approximately 1 x 10⁶ recombinant clones of a Igt10 human fetal brain cDNA library (CLONTECH) were plated and screened following standard techniques (Sambrook et al., 1982). Library screening was performed using the IMAGE consortium cDNA clones *44328* (ESTs *H05397* ), 565548 (ESTAA*129714*) and further positive clones. Positive phage were plaque purified and their inserts were amplified by PCR using Igt10 primers flanking the cloning site. All inserts were digested with MboI and AluI to generate a consensus restriction enzyme map. Direct sequencing with Igt10 primers was also performed using purified inserts as templates.

### d) Genomic DNA analysis of human and mouse cosmid and phage clones

Human cosmid clones were identified by screening the ICRF flow-sorted human X chromosome library (Lehrach, H., et al., 1990) with ESTs *565548* and *44328*, and obtained from the german resource center (RZPD). As no positive cosmid clones corresponding to the 5' end of the gene was identified in the cosmid library, the YAC clone *737H4* was subcloned into EMBL3 phage. MboI partial digestion and EMBL3 BamHI digested arms were used to construct the library. Screening of the library with the cDNA inserts and purification of phage DNA corresponding to positive clones were performed according to standard procedures (Sambrook et al., 1982). HindIII digested phage DNA was subcloned into pBluescript SK(+) vector. Mouse phage clones were isolated by screening mouse genomic phage library (genomic DNA of 729 strain). Screening was performed at a low stringency using cDNA 1C. HindIII digested DNA from positive clones were subcloned into pBluescript (+). Subclones corresponding to the 5' end of the gene were sequenced with T3 and T7 primers, and with human exonic primers.

### 2. Results :

### a) YAC contig of the XLIS critical region and physical mapping of ESTs

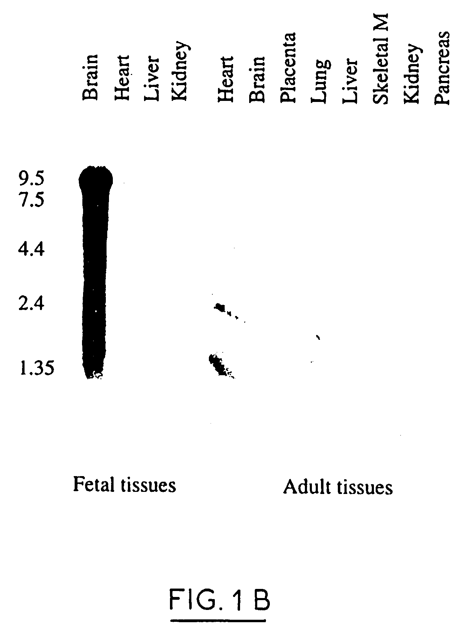

Des Portes et al., (1997) identified *DXS1072* as the distal recombinant marker of the *XLIS* genetic locus ; also Ross et al., (1997) mapped the breakpoint of the (X;2) translocation associated with lissencephaly, distal to *DXS287*. Therefore, the critical region of the *XLIS* gene was identified, extending from *DXS287* to *DXS1072*. To generate a YAC contig covering the region of interest, data available in the Whitehead Institute/MIT Center data base were used as a basis and YAC clones previously localized within bands Xq22.3-q23 from *DSX1210* to *DXS1072* were requested. Overlaps between clones were analysed by PCR amplification of fourteen STS and hybridization of HindIII digested YAC DNA blots, using STSs as probes. Thus, a reliable contig, with only one gap between contigs wcx-27 and wc-769 was constructed (figure 1a). Fifteen ESTs roughly localized on radiation hybrid pannels within the Xq22.2-q24 region by the Human Gene Map consortium (Schuler et al., 1996) were fine mapped by PCR amplification and hybridization on the YAC contig. Only eight ESTs were localized on the constructed YAC contig. Their expression was studied by hybridization of EST probes to fetal and adults multiple tissue Northern blots. One EST (*SGC34529*), showed a strong signal, corresponding to a 9.5 kb long transcript and present only in fetal brain (figure 1b). The localization of this EST in the region of interest (distal to *DXS287* and proximal to *DXS1072*), and its high level of expression in fetal brain led the present inventors to consider the gene corresponding to this EST as a candidate for X-SCLH/LIS condition.

### b) Isolation and characterisation of the full length (9.5 kb) candidate transcript

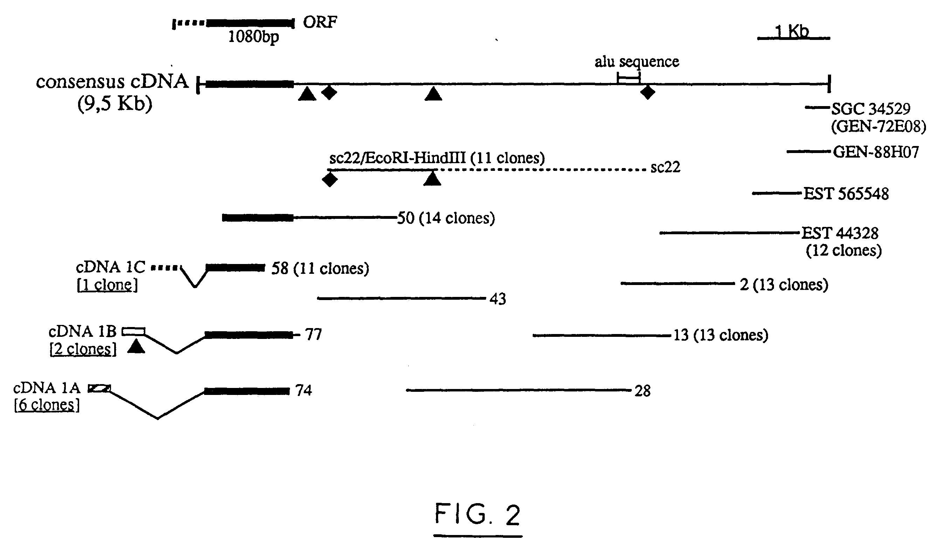

Taking into account overlapping EST sequences available in GenBank data bases (figure 2), a preliminary cDNA contig (2.5 kb long) was set up. Then, two clones of this contig (ESTs *565548* and *44328*) were used to screen a human fetal brain cDNA library. Seven walks were required to clone the full length transcript (figure 2). At each screening, inserts of purified positive clones were amplified by PCR and their ends sequenced. The first three walks and sequences of the corresponding cDNA clones did not allow to identify any potential ORF; in addition, the presence of an Alu sequence in several cDNA clones and the colinearity between the consensus sequence of the cDNA and genomic DNA sequence, assessed by hybridization and sequence identity, suggested a large 3' untranslated region. This latter region is included within the HindIII fragment of about 9 kb and the overlapping EcoRI sc22 genomic fragments (figure 2 and 3b). These fragments were generated from a cosmid clone (ICRF coordinates: c104J0516Q8 , also called cosmid 9 in figure 3b) which was isolated from the flow sorted human X-specific cosmid library by ESTs *565548* and *44328*. It was then decided to use the genomic EcoRI/HindIII fragment of the *sc22* subclone to screen the cDNA library which enabled us to reach the coding part of the cDNA (figure 2). Localisation in Xq22 critical region of the cDNA and genomic fragments was performed by hybridizations on Southern blots containing HindIII digested DNA of YAC clones covering the critical region, and of two different somatic hybrids containing either the whole human X chromosome or a translocated der12 chromosome derived from an (X;12)(q11;q15) translocation (Bienvenu et al., 1997) containing therefore the region of interest. At each walk in the cDNA library, and after confirmation of the overlapping between the clone used as a probe and the new clones, at least the insert of one new clone is used to probe the above described Southern blots and the fetal and adult multiple tissue Northern blots.

The sequence of both ends of the large number of positive clones obtained after each screening of the cDNA library (52 clones in total) allowed the inventors to generate 85 kb of sequence and a reliable consensus sequence of about 9.5 kb representing the full-lengh cDNA, bypassing therefore any further subcloning of cDNA clones. The consensus sequence of the cDNA showed a single open-reading frame ORF of 1080 bp starting from a putative translation initiation codon (CAAAAT**ATG**G) in good agreement with the Kozak consensus sequence (Kozak., 1986). This ORF encodes a predicted protein of 360 amino acids. Sequence analysis of the cDNA clones corresponding to the 5' end of the transcript showed three divergent types of sequences: cDNA 1A (8 clones), cDNA 1B (2 clones) and cDNA 1C (1 clone) (figure 2). The sequence of cDNA 1C (represented by only one clone) showed an additional ORF encoding for 42 amino-acids which are in frame with the downstream ATG (figure 2 and 3a). This additional in frame ORF starts also at an ATG flanked by a good consensus sequence (Fig 3a). In order to define the genomic structure of this gene, the present inventors constructed and investigated a cosmid/phage contig that covers the gene (figure 3b). Determination of exon-intron boundaries was performed through sequence comparison between cDNA clones and genomic DNA, which led to the identification of 9 exons (figure 3b). The common ORF is encoded by exon 2 to exon 6 and the initial 54 nucleotides of the last exon. The identified splice junction sequences (data not shown) exhibit close adherence to the 5' and 3' consensus sequences (Senapathy et al.,1990).

The structure of this gene is unusual in that only 16% of its sequence is coding and the 3' UTR, which is contained in only one exon, is 7.9 kb long. The extensive 3'UTR contains two AU-rich elements (AREs), defined by AUUUA motifs which are present in the 3'UTR of many labile mRNAs thought to be involved in the regulation of mRNA stability (McCarty and Kollmus, 1995).

In order to clarify the divergence of the 5' end sequences, the present inventors cloned the corresponding genomic region, characterized three exons 1A, 1B and 1C at the 5' end of the candidate gene and performed RT-PCR experiments using different combinations of primers (figure 3b). The absence of consensus splice site at the 5' end of exon 1A suggested that this exon corresponds to the 5' end of the 1A-transcript isoform. In line with this hypothesis is the presence in the genomic sequence upstream from exon 1A of a TATA box, 2 AP1 (Boyle et al., 1991) and 2 brn2/N-Oct3 (Li et al., 1993) consensus putative binding sites, reminescent of a promoter region (data not shown). It is worth noting that N-Oct3 is a highly expressed CNS specific POU domain transcription factor (Schreiber et al., 1993). Results of RT-PCR experiments using human fetal brain RNA (at 21 weeks of gestational age) are represented in Figure 3b. In addition to the alternative splicing event concerning exon 1C (isoforms 1 and 3), it appears that exon 1B, which has a potential splice acceptor site, is spliced neither with 1C nor with 1A. Only RT-PCR products resulting from a splicing with the first common exon were obtained (figure 3b, isoform 2), suggesting that transcripts containing exon 1B are expressed from a potential alternative promoter. These data suggest that alternative splicing events and potential alternative start sites of transcription are involved in the diversity of transcripts produced by this gene.

### EXAMPLE 2: Identification of mutations in unrelated patients with X-SCLH/LIS syndrome

### A - IDENTIFICATION OF MUTATIONS BY RT-PCR

### 1. Experimental procedure

Total RNA was extracted from EBV-transformed lymphoblastoid cell lines by the guanidium thiocyanate method using the RNA-B™ extraction kit (Bioprobe systems). First strand synthesis of cDNA using 2 µg of total RNA was carried out in a final volume of 40 µl according to a standard procedure. 40 cycles of PCR were performed (94°C, 30s; 55°C, 30s; 72°C, 1 min, in a PTC200 MJ Reseach machine) on 5 µl of the cDNA sample using one of four sets of primers (figure 4) to obtain four overlapping fragments spanning the whole *XLIS* coding sequence. Then a second round of PCR amplification with nested primers (figure 4) was performed using 0.5 µl of the first PCR product. Both strands of nested PCR products were directy sequenced using the DyeDeoxy terminator cycle sequencing kit protocol (Applied Biosystems). Cosegregation of mutations with phenotypes were carried out on genomic DNA using appropriate restriction enzymes (figure 5).

### 2. Results

To prove that the isolated gene is reponsible for X-SCLH/LIS syndrome, five unrelated individuals were analyzed for the presence of mutations: affected males of three previously mapped X-SCLH/LIS families: 2 caucasian and one black from Guadeloupe, (des Portes et al., 1997) and two caucasian sporadic female cases, patient OD with SCLH and patient MJ with pachygyria and corpus collosum agenesia. The strategy involved amplification by nested RT-PCR (figure 4) and direct sequencing of the few copies, also called illegitimate (Chelly et al., 1989) or ectopic (Sarkar et al., 1989), of the *XLIS* candidate transcript present in total lymphoblastoid cell line RNAs. The complete coding sequence was sequenced on both strands in the 5 patients.

The sizes of nested PCR products analysed on 2 % Nusieve gel were normal in all patients except patient MJ. In this patient, analysis of fragment 3 revealed an additional band shorter than the 375 bp expected length. Sequence analysis of the abnormal cDNA fragment showed a deletion of 103 bp, corresponding to the complete exon 4 (figure 3b), also called exon H in figure 5d. The cause of this abnormal exon skipping was identified by the analysis of the genomic sequences flanking the skipped exon which revealed an heterozygous point mutation, GT to AT, at the invariant dinucleotide GT of the 5' donor site (figure 5d). This exon skipping causes a frameshift and premature termination 4 residues downstream of the aberrant splicing (figure 5d). This splice site mutation is a new mutation as it was not found in genomic DNA of the two healthy parents.

The nucleotide sequence of the three familial cases revealed the presence of independent missense point mutations. However, no sequence abnormality was detected in the remaining sporadic case of SCLH (patient DO). Pedigrees, MRI images and corresponding mutations are shown in figures 5a, b and c. Positions of the mutations are summarized in Table 2.

**Table 2**

| Summary of mutations in *XLIS*/SCLH patients | | | |
|---|---|---|---|
| Patient | Type of mutation | Nucleotide position | Effect of mutation |
| Family 1 | G to A | 599 | asp to asn |
| Family 2 | C to T | 989 | arg to trp |
| Family 3 | T to C | 788 | tyr to his |
| JM case (sporadic) | G to A donor splice site | exon-intron junction +1 from 1223 | aberrant splicing, frameshift and stop codon 1236 |

Amino acid substitutions generated by these missense mutations change either the neutral-polar or acid-base nature of the amino acid residues involved. Cosegregation of the mutations with the disease was confirmed in all three families on genomic DNA using either restriction enzymes (families 1 and 2 showed in figure 5a and 5b) or allele specific amplification (family 3 showed in figure 5c). In the latter family, identification of the mutation allowed to reassess the genotype of all members of the large pedigree as reported in des Portes et al. (1997), and excluded the involvement of the mutation in the ambiguous brain MRI abnormalities observed in two females (cousins of affected cases). The presence of the four mutations (missense mutations and splice mutation) was systematically tested in a control population: none of the mutations was detected among 100 control X chromosomes. Control individuals are mainly (90%) of caucasian origin.

### B - IDENTIFICATION OF MUTATIONS BY DGGE

The authors of the present invention implemented the DGGE method for mutations screening of thirteen unrelated SCLH genomic DNAs. Cases were studied the phenotype of each family member and routine MRI or CT scans were checked by the same pediatric neurologist.

The DGGE method was carried out according to the standard procedure known by one skilled in the art.

The parameters for amplification of the *XLIS* gene fragments and DGGE conditions are reported in table 3:

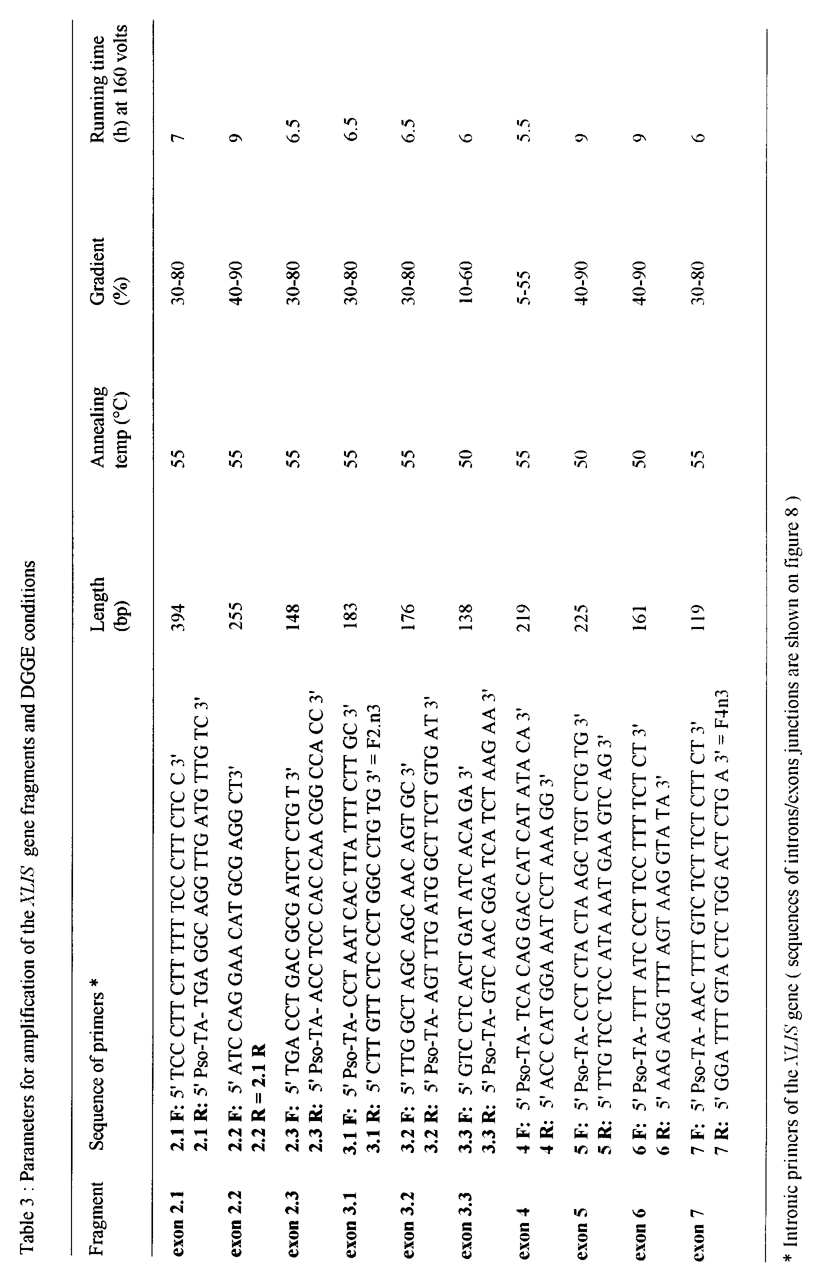

In addition to the 4 mutations reported in Example 2A, a variety of mutations was found in 7 out of the 13 new explored cases. Among these 7 new cases with a mutated *XLIS* gene, three patients had null mutations, either nonsense point mutations or aberrant splicing leading to premature stop codon. In the four other cases, including a familial case, missense mutations leading to drastic amino acid substitution were detected. Each of the missense mutations cosegregated with the phenotype and none was found in hundred control chromosomes, ruling out common polymorphisms.

The clinical severity of SCLH varies strikingly from asymptomatic MRI heterotopic bands to severe mental impairment with untractable epilepsy. The relative thickness of the heterotopic band correlates with the phenotype as patients with thicker bands have more severe mental retardation and seizures (Raymond et al., 1995 ; Barkovich et al., 1994). Furthermore, a SCLH "forme fruste" consisting of bilateral and symetric bands with a regional distribution has been described (Franzoni et al., 1995).The present data may suggest a correlation between the clinical severity and mutation profiles. Indeed, the four null mutations (nonsenses and aberrant splices with premature stop codon) occur in severely affected females with thick SCLH or pachygyria.

### EXAMPLE 3: Expression of the XLIS gene

### 1. Experimental procedure

Fetal and adult mutiple-tissue Northern blots (Clontech) were hybridized with ESTs and cDNA clones and subsequently washed according to standard procedures. For RT-PCR experiments, total RNA samples were prepared from human fetal brain (21 weeks old), embryonic (E15), newborn and postnatal (P60) mouse brains. Cells were derived from brains of random-bred Swiss mice. Glial cells were from newborn mouse cerebral hemispheres. Ninety-five per cent of the cells were identified as type-1 astrocytes; neither neurons nor oligodendrocytes were detected in multiple screenings. Culture of neuronal cells were set up from single-cell suspension of fetal brains at 15 days of gestation. Cultures consisted predominantly of neurons (> 95 %), identified by surface labelling with tetanus toxin and intracellular labelling with antibodies to g-enolase or neurofilament proteins. Amplification by RT-PCR was performed according to standard procedure. Products obtained after 25 and 35 cycles of PCR were analysed by gel electrophoresis and ethidium bromid staining. Primer sequences used for RT-PCR are described in figure legends (figure 6h).

For *in situ* hybridization, 8mm thick coronal sections of fetal brain were fixed in 4% (w/v) paraformaldehyde, cryoprotected with 10% sucrose in phosphate buffer, freezed with isopenthan and stored at -80°C until sectionning. Briefly, hybridization of coronal brain sections (10-14 µm thickness) with sense and antisense α³⁵S-labelled RNA probes was carried out in a 50% formamide solution at 52°C. Sections were successively washed in 50% formamide, processed for digestion with RNase A and T1, and washed by successive passages in progessive stringent solutions. Final washing conditions are 0.1SSC solution at 60°C. Sections were first exposed for three days in cassette with autoradiographic film, then slides were dipped in dilueted Kodak NTB2 emulsion and exposed for 5 to 15 days. Emulsion autoradiographs were developed and sections were counterstained with toluidine blue, mounted in Eukitt and examined under light microscope.

### 2. Results

As shown in figure 2, a large and highly expressed *XLIS* transcript of about 9.5 kb was detected only in fetal brain, but not in other tested tissues. As X-SCLH/LIS syndrome is believed to result from an arrest of neuronal migration*, in situ* hybridization was used to examine *XLIS* expression in developing human cerebral cortex. Coronal sections of a human cerebral cortex at 27 weeks of gestational age were hybridized with *XLIS* antisense (fig 6b) and sense (fig 6a) probes. Autoradiograms suggest a strong labelling of the ventricular zone (VZ) and cortical plate (CP), and a moderate labelling of the intermediate zone (IZ). At higher magnification, it appears that *XLIS* is expressed in the majority of cells of the CP, IZ and VZ. Rare negative cells were identified in the three zone. In the IZ, labelled cells are organized as oriented chains (fig 6f) reminiscent of migrating neurons.

In order to confirm the expression in neuronal cells, the present inventors cloned the mouse homologous gene, *xlis*, derived appropriate primers (1AF and CoR primers as described in legend to figure 6h) and investigated by RT-PCR the expression of *xlis*, (i) in mouse brain at embryonic day (E) 15, postnatal days (P) 1 and 60, and (ii) in primary cultures of mouse neuronal and astro-glial cells derived from fetal brains at E15 and newborn mouse brains, respectively. Figure 6h shows the results after 25 cycles of RT-PCR amplification of the *xlis* mRNA and mouse *GDI-1* mRNA (*rab GDP*-*dissociation inhibitor*, Shisheva et al., 1994) known to be expressed in developping brain (Bächner et al., 1995), and used here as a control. In addition to the expected decrease of *xlis* expression after birth, figure 6h shows that in primary cultures of neuronal cells a significant level of *xlis* expression is observed whereas it is not detected in glial cells. This latter result was also obtained after 35 cycles of amplification.

These results indicate that *XLIS* gene is mainly expressed during early brain development in neuronal cells including VZ precursors and migrating neurons.

### EXAMPLE 4 : XLIS gene encodes a novel polypeptide: doublecortin

The open reading frame starting at the ATG common to all types of transcript encodes a predicted protein of 360 amino acids named doublecortin (figure 3a). However, if the alternatively spliced in-frame exon 1C is taken into account, the *XLIS* gene product would be composed of 402 amino acids. Hydropathicity analysis (Kyte and Doolite, 1982) of the deduced amino acid sequence did not reveal the presence of either signal peptide or hydrophobic segment reminescent of transmembrane domains and suggested that doublecortin is hydrophilic and probably intracellular. Based on consensus protein kinase phosphorylation site motifs (Kemp and Prearson, 1990; Songyang et al., 1995), several potential phosphorylation sites for protein kinase C and casein kinase II and one potential site for AbI at tyrosine residue 70 were identified in the deduced protein (see figure 3a). Comparison with nucleotide and protein sequences in data bases using BLAST and FASTA, indicated that doublecortin has no significant homology to any protein of known function, except with a gene product of 729 amino acids (GeneBank accession number AB002367, gene called AA0369) reported in a recent large scale study of brain cDNA clones (Nagase et al., 1997). This similarity of about 75 % starts at the N-terminal end of both proteins and extends over 340 amino acids (figure 7). It is noteworthy that BLAST searches concerning the remaining C-terminal part of the 729 amino acids protein showed a significant homology with calcium calmodulin-dependent (CaM) kinases type II. The highest score, 97% identity, was observed with the rattus norvegicus CaM-kinase cpg16 (Hevroni, GenBank accession number U78857). These data suggest that the polypeptide of 729 amino acids named *XLIS homologous*, has two major segments: an N-terminal domain of about 340 amino acids homologous to doublecortin and a C-terminal part of 389 amino acids bearing an extensive homology with protein kinases.

Doublecortin also showed homology over a short segment of 30 amino acids (position 312 to 342) with the N-terminal domain (position 8 to 38) of the rattus norvegicus CaM-kinase cpg16 (figure 7).

The expression of *XLIS homologous* gene (AA0369) was analysed by Northern blot hybridization and showed the presence of a major transcript of about 7.5 kb expressed only in fetal brain with a persistent expression, but at lower levels, in adult brain.

### REFERENCES

Aicardi, J. (1991). The agyria-pachygyria complex: a spectrum of cortical malformations. Brain Dev *13*, 1-8.
Allendoerfer, K. L., and Shatz, C. J. (1994). The subplate, a transient cortical structure: its role in the development of connections between thalamus and cortex. ann. Rev. Neurosci. *17*, 185-218.
Antonarakis SE. 1989, Diagnosis of genetic disorders at the DNA level. N Engl J. Med. 320: 153-163.
Bächner, D., Sedlacek, Z., Korn, B., Hameister, H., and Poustka, A. (1995). Expression patterns of two human genes coding for different rab GDP-dissociation inhibitors (GDIs), extremely conserved proteins involved in cellular transport. Hum. Mol. Genet. *4*, 701-708.
Barkovich, A., Guerrini, R., Battaglia, G. Kalifa, G., N'guyen, T., Parmeggiani, A., Santucci, M., Giovanardi-Rossi, P., Granata, T., D'Incerti, L. Band heterotopia: correlation of outcome with magnetic resonance imaging parameters. (1994) Ann Neurol .36, 609-617.
Bellis, M. and Casellas P., médecine/sciences, (1997) 13 : 1317-24.
Berry-Kravis, E., Israel, J. X-linked pachygyria and agenesis of the corpus callosum: evidence for an X chromosome lissencephaly locus. (1994)Ann Neurol. 36, 229-233.
Bienvenu, T., Der-Sakissian, H., Billuart, P., Tissot, M., des Portes, V., Brüls, T., Chabrolle, J. P., Chauveau, P., Cherry, M., Kahn, A., Cohen, D., Beldjord, C., Chelly, J., and Cherif D. (1997). Mapping of the X-breakpoint involved in a balanced X; 12 translocation in a female with mild mental retardation. Eur. J. Hum. Genet. 5, 105-109.
Boyle, W., Smeal, T., Defize, L., Karin, M, and Hunter, T. (1991). Activation of protein kinase C decreases posphorylation of c-Jun at sites that negatively regulate its DNA-binding activity. Cell *64*, 573-584.
Chelly, J., Concordet, J. P., Kaplan, J. C., and Kahn, A. (1989). Illegitimate transcription: Transcription of any gene in any cell type. Proc. Natl. Acad. Sci. USA *86*, 2617-2621.
Cooper DN, Schmidtke J., 1991, Diagnosis of genetic disease using recombinant DNA, 3^{rd} Edition, Hum Genet., 87: 519-560.
des Portes, V., Pinard, J.M., Smadja, D., Motte, J., Boespflüg-Tanguy, O., Moutard, M.L., Desguerre, I., Billuart, P., Carrie, A., Bienvenu, T., Vinet, M.C., Bachner, L., Beldjord, C., Dulac, O., Kahn, A., Ponsot, G., and J. Chelly. (1997). Dominant X-linked subcortical laminar heterotopia and lissencephaly syndrome (X-SCLH/LIS): evidence for the occurence of mutation in male and mapping of a potential locus in Xq22. J. Med. Genet. 34, 177-183.
Dib, C., Fauré, S., Fizames, C., Samson, D., Drouot, N., Vignal, A., Millasseau, P., Marc, S., Hazan, J., Seboun, E., et al. (1996). A comprehensive genetic map of the human genome based on 5.264 microsatellites. Nature *380*, 152-154.
Dobyns, W., Andermann, E., Andrermann, F., Czapansky-Beilman, D., Dubeau, F., Dulac, O., Guerrini, R., Hirsch, B., Ledbetter, D., Lee, N., Motte, J., Pinard, J. M., Radtke, R., Ross, M., Tampieri, D., Walsh, C., and Truwit, C. (7996). X-linked malformations of neuronal migration. Neurology *47*, 331-339.
Franzoni, E., Bernardi, B., Marchiarni, V., Crisanti, A., Marchi, R., Fonda, C. (1995) Band brain heterotopia. Case report and literature review. Neuropediatrics 26, 37-40.
Harding, B. (1996). Gray Matter Heterotopia. In Dysplasias of cerebral Cortex and Epilepsy, R. Guerrini, F. Andermann, R. Canapicchi, J. Roger, B. Zilfkin, and P. Pfanner, eds. (Philadelphia-New York : Lippincott-Raven), pp. 81-88.
Houdou, S., Kuruta, H., Konomi, H., and Takashima, S. (1990). Structure in lissencephaly determined by immunohistochemical staining. Pediatr. Neurol. 6, 402-406.
Howell, B. W., Hawkes, R., Soriano, P., and Cooper, J., A. (1997). Neuronal position in the developping brain is regulated by mouse disabled-1. Nature. *389*, 733-737.
Huttenlocher, A., Sandborg, R., and Horwitz, A. (1995). Adhesion in cell migration. Curr. Opin. Cell Biol. 7, 697-706.
Hynes, R. O., and Lander, A. D. (1992). Contact and adhesive specificities in the associations, migrations, and targeting of cells and axons. Cell *68*, 303-322.
Kemp, B. E., and Pearson, R. B. (1990). Protein kinase recognition sequence motif. Trends Biochem. Sci. *15*, 342-346.
Köhler and Milstein, Nature (1995), vol. *256*, 495-497.
Komuro, H., and Rakic, P. (1992). Selective role of N-type calcium channels in neuronal migration. Science *257*, 806-809.
Kozak, M. (1986). Point mutations define a sequence flanking the AUG initiator codon that modulates translation by eukaryotic ribosomes. Cell *44*, 283-292.
Kuzniecky, R., Murro, A., King D. et al. (1993). Magnetic resonance imaging in childhood intractable partial epilepsy: pathologic correlations. Neurology *43*, 681-687.
Kyte, J., and Doolittle, R. F. (1982). A simple method for displaying the hydropathic character of a protein. J. Mol. Biol. *157*, 105-132.
Lehrach, H., et al. (1990). In Genome analysis Volume 1: Genetic and physical mapping, K. E. Davies and S. M. Tilghman, eds. (Cold spring Harbor Laboratory Press, Cold Spring Harbor), pp. 39-81.
Li, P., He, X., Gerrero, M. R., Mok, M., Aggarwal, A., and Rosenfeld, M. G. (1993). Spacing and orientation of bipartite DNA-binding motifs as potential functional determinants for POU domain factors. Genes Dev. 7, 2483-2496.
Lo Nigro, C., Chong, S., Smith, A.C., Dobyns, W. B., Carrozzo, R., and Ledbetter, D. H. (1997). Point mutations and intragenic deletion in LIS1, the lissencephaly causative gene in isolated lissencephaly sequence and Miller-Dieker syndrome. Hum. Mol. Genet. *6*, 157-164.
Luskin, M. B., Pearlman, A. L., and Sanes, J. R. (1988). Cell lineage in the cerebral cortex of the mouse studied in vivo and in vitro with a recombinant retrovirus. Neuron *1*, 653-647.
McCarthy, J. E., and Kollmus, H. (1995). Cytoplasmic mRNA-protein interactions in eukaryotic gene expression. Trens Biochem. Sci. *20*, 191-197.
McConnell, S. K. (1995). Constructing the cerebral cortex: neurogenesis and fate determination. Neuron *15*, 761-768.
Nagase, T., Ishikawa, K., Nakajima, D., Ohira, M., Seki, N., Miyajima N., Tanaka, A., Kotani, H., Nomura, N. and Ohara, O. (1997). Prediction of the coding sequences of unidentified human genes. VII. The complete sequences of 100 new cDNA clones from brain which can code for large proteins in vitro. DNA Res. *4*, 141-150.
O'Rourke, N. A., Sullivan, D. P., Kasnowski, C. E., Jacobs, A. A. and McConnell, S. K. (1995). Tangential migration of neurons in the developing cerebral cortex. Development *121*, 2165-2176.
Palmini, A., Andermann, F., Aicardi, J., Dulac, O., Chaves, F., Ponsot, G., Pinard, J-M., Goutières, F., Livingston, J., Tampieri, D., Andermann, E,. and Robitaille, Y. (1991). Diffuse cortical dysplasia, or the "double cortex" syndrome: the clinical and epileptic spectrum in 10 patients. Neurology *41*, 1656-1662.
Pinard, J-M., Motte, J., Chiron, C., Brian, R., Andermann, E., and Dulac, O. (1994). Subcortical laminar heterotopia and lissencephaly in two families: a single X linked dominant gene. J Neurol. Neurosurg. Psychiatry 57, 914-920.
Rakic, P. (7972). Mode of cell migration to the superficial layers of fetal monkey neocortex. J. Comp. Neurol *145*, 61-84.
Rakic, P. (1988). Specification of cerebral cortical areas. Science *241*, 170-176.
Rakic, P., and Caviness, V., S., Jr. (1995). Cortical development: view from neurological mutants two decades later. Neuron *14*, 1101-1104.
Raymond, A., Fish, D., Sisodiya, S., Alsanjari, N., Stevens J., Shorvon, S. (1995). Abnomalities of gyration, heterotopias, tuberous sclerosis, focal cortical dysplasia, microdysgenesis, dysembryoplastic neuroepithelial tumor and dysgenesis of the archicortex in epilepsy. Clinical, EEG and neuroimaging features in 100 adult patients. Brain *118*, 629-660.
Ross, E., Allen, K., Srivastava, A., Featherstone, T., Gleeson, J., Hirsch, B., Harding, B., Andermann, E., Abdullah, R., Berg, M., Czapansky-Bielman, D., et al. (1997). Linkage and physical mapping of X-linked lissencephaly/SBH (*XLIS*): a gene causing neuronal migration defects in human brain. Hum. Mol. Genet. *6*, 555-562.
Sambrook, J., Fritsch, E., Maniatis, T. C. (1989), In Molecular cloning: a laboratory manual, (Cold spring Harbor Laboratory Press).
Sarkar, G., and Sommer, S. (1989). Access to a messenger or its protein product is not limited by tissue or species specificity. Science *244,* 331 - 334.
Schreiber, E., Tobler, A., Malipiero, U., Schaffner, W., and Fontana, A. (1993). cDNA cloning of human N-Oct 3, a nervous-system specific POU domain transcription factor binding to the octamer DNA motif. Nucl. Ac. Res. *21*, 253-258.
Schuler, G.D., Boguski, M. S., Stewart, E. A., Stein, L. D., Gyapay, G., Rice, K., White, R. E., Rodriguez-Tomé, P., Aggarwal, A., Bajorek, E., et al. (1996). A gene map of the human genome. Science *274*, 540-546.
Senapathy, P., Shapiro, M. B., and Harris, N. L. (1990). Splice junction, branch point sites, and exons: sequence statistics, identification, and application to the genome project. Meth. Enzymol. *183*, 252-278.
Shisheva, A., Südhof, T., and Czech, P. (1994). Cloning, characterization, and expression of a novel GDP dissociation inhibitor isoform from skeletal muscle. Mol. Cell. Biol. *14*, 3459-3468.
Sheldon, M., Rice, D. S., D'Arcangelo, G., Yoneshima, H., Nakajima, K., Mikoshiba, K., Howell, B. W., Cooper, J. A., Goldowitz, D., and Curran, T. (*1997*). *Scrambler* and *yotari* disrupt the *disabled* gene and produce a *reeler*-like phenotype in mice. Nature. *389*, 730-733.
Songyang, Z., Carraway III, K. L., Eck, M. J., Harrioson, S. C., Feldman, R. A., Mohammadi, M., Schlessinger, J., Hubbard, S. R., Smith, D. P., Eng, C., Lorenzo, M. J., Ponder, B. A. J., Mayer, B. J., and Cantley, L. C. (1995). Catalytic specificity of protein-tyrosine kinases is critical for selective signaling. Nature. 373,536-539.
Tan, S. S. and Breen, S. (1993). Radial mosaicism and tangential cell dispersion both contribute to mouse neocortical development. Nature 362, 638-640.
Walsh, C., and Cepko, C. L. (1992). Widespread dispersion of neuronal clones across functional regions of the cerebral cortex. Science 255, 434-440.
Ware, M. L., Fox, J. W., Gonzalez, J. L., Davis, N. M., de Rouvroit, C. L., Russo, C. J., Chua, S. C., Goffinet, Jr., A. M., and Walsh, C. A. (1997). Aberrant splicing of a mouse disabled homolog, mdab1, in the scrambler mouse. Neuron *19*, 239-249.
Zollino, M., Mastroiacovo, P., Zampino, G., Mariotti, P., Neri, G. (1992) New XLMR syndrome with characterisyic face, hypogenitalism, congenital hypotonia and pachygyria. *Am J Med Genet*. **43**, 452-7.

## Claims

1. Isolated nucleic acid sequence selected from the group consisting of SEQ ID n° 1 to SEQ ID n° 9, a derivative nucleic acid sequence thereof and a homologous nucleic acid sequence thereof.

2. Isolated nucleic acid sequence selected from the group consisting of SEQ ID n° 70 to SEQ ID n° 20, a derivative nucleic acid sequence thereof and a homologous sequence thereof.

3. Isolated nucleic acid sequence, said sequence differing from said nucleic acid sequences of any of claims 1 or 2 by one or more mutation(s) selected from the mutations defined in table 1.

4. Isolated *XLIS* polypeptide substantially having the aminoacid sequence encoded by a nucleic acid sequence of claim 2.

5. Isolated *XLIS* polypeptide of claim 4 wherein said aminoacid sequence is selected from the group consisting of SEQ ID n° 21, SEQ ID n° 22, and a derivative amino acid sequence thereof.

6. Vector for cloning and/or expression comprising a nucleic acid sequence of any of claims 1 and 2.

7. Host cell transfected with a vector according to claim 6.

8. Nucleic acid sequence which specifically hybridizes with a nucleic acid sequence according to any of claims 1 and 2.

9. Nucleic acid sequence of claim 8 selected from the group consisting of SEQ ID n° 23 to SEQ ID n° 66.

10. Method for producing a recombining *XLIS* polypeptide, wherein a host cell of claim 7 is transfected with a vector of claim 6 and is cultured in conditions allowing the expression of a polypeptide according to any of claims 4 and 5.

11. Monoclonal or polyclonal antibodies, or fragments thereof, chimeric or immunoconjugate antibobies, which are capable of specifically recognizing a polypeptide according to any of claims 4 and 5.

12. Use of the antibodies of claim 11 for detecting or purifiyng a polypeptide according to any of claims 4 and 5 in a biological sample.

13. Use of a nucleic acid sequence according to any of claims 1, 2, 3, 6 and 7, for detecting an abnormality in the *XLIS* gene or in the transcripts of the *XLIS* gene.

14. Method of *in vitro* diagnosis of a neurological disorder associated with an abnormality in the *XLIS* gene or in the transcripts of the *XLIS* gene, wherein one or more mutation(s) is detected in the *XLIS* gene or in the transcripts of the *XLIS* gene.

15. Method according to claim 14 wherein said mutations are selected from the mutations defined in table 1.

16. Method of *in vitro* diagnosis according to any of claims 14 or 15 comprising the steps of:
- contacting a biological sample containing DNA with specific oligonucleotides permitting the amplification of all or part of the *XLIS* gene, the DNA contained in the sample having being rendered accessible, where appropriate, to hybridization, and under conditions permitting a hybridization of the primers with the DNA contained in the biological sample;
- amplifying said DNA;
- detecting the amplification products;
- comparing the amplified products as obtained to the amplified products obtained with a normal control biological sample, and thereby detecting a possible abnormality in the *XLIS* gene.

17. Method of *in vitro* diagnosis according to any of claims 14 or 15 comprising the steps of:
- producing cDNA from mRNA contained in a biological sample ;
- contacting said cDNA with specific oligonucleotides permitting the amplification of all or part of the transcript of the *XLIS* gene, under conditions permitting a hybridization of the primers with said cDNA;
- amplifying said cDNA;
- detecting the amplification products;
- comparing the amplified products as obtained to the amplified products obtained with a normal control biological sample, and thereby detecting a possible abnormality in the transcript of the *XLIS* gene.

18. Pharmaceutical composition comprising a purified doublecortin polypeptide of the invention and/or a homologous polypeptide thereof, in association with a pharmaceutically acceptable carrier.
